# EUROPÄISCHE PATENTANMELDUNG

(11) **EP 2 865 676 A1**
(43) Veröffentlichungstag der Anmeldung: **29.04.2015**
(21) Anmeldenummer: 14164580.4
(22) Anmeldetag: 14.04.2014
(51) Int. Cl.: C07D 309/04

(54) **Herstellung von 2-substituierten 4-Methyl-tetrahydropyranen aus 2-Alkyl-4,4-dimethyl-1,3-dioxan-haltigen Ausgangsstoffen**

(71) Anmelder: BASF SE, 67056 Ludwigshafen (DE)
(72) Erfinder: Rüdenauer, Stefan, 69469 Weinheim (DE); Stork, Timon, 68642 Bürstadt Bobstadt (DE)
(74) Vertreter: Reitstötter Kinzebach

(57) **Zusammenfassung**

Die vorliegende Erfindung betrifft ein Verfahren zur Herstellung von 2-substituierten 4-Methyl-tetrahydropyranen der allgemeinen Formel (I) 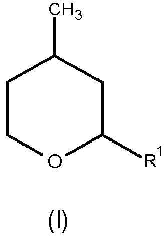 aus Ausgangsmaterialien, die wenigstens ein 4,4-Dimethyl-1,3-dioxan der allgemeinen Formel (II) enthalten.

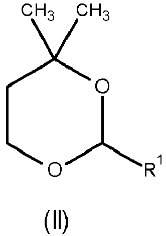

## Beschreibung

### HINTERGRUND DER ERFINDUNG

Die vorliegende Erfindung betrifft ein Verfahren zur Herstellung von 2-substituierten 4-Methyl-tetrahydropyranen aus Ausgangsmaterialien, die wenigstens ein 2-Alkyl-4,4-dimethyl-1,3-dioxan enthalten.

### STAND DER TECHNIK

Alkylsubstituierte Tetrahydropyrane haben weite Verbreitung als Aroma- und Geschmacksstoffe gefunden. Ein bekannter Vertreter aus dieser Klasse ist das 2-(2-Methyl-1-propenyl)-4-methyltetrahydropyran (Rosenoxid), welches eine erfrischende, blumige und frische Duftnote aufweist. Der Duft variiert dabei in Abhängigkeit von den eingesetzten Isomeren, wobei jedes Isomer über eine charakteristische Note verfügt.

Aufgrund seiner eingeschränkten Stabilität, der begrenzten Verfügbarkeit von natürlichem Rosenoxid bzw. der aufwändigen und somit teuren Synthese wurde nach günstigeren Alternativen mit vergleichbaren olfaktorischen Eigenschaften gesucht. Eine solche Alternative ist das 2-(2-Methylpropyl)-4-methyl-tetrahydro-2H-pyran, das auch als Dihydrorosenoxid bezeichnet wird.

Die Erstsynthese von Dihydrorosenoxid wurde von M. Julia und B. Jacquet in Bulletin de la Societe Chimique de France 1963, 8-9, 1983, beschrieben. Ausgehend von But-2-en-1-al wurde durch eine Diels-Alder-Reaktion mit Ethylvinylether und anschließender Hydrierung ein cyclisches Acetal erhalten. Nach Abspaltung von Ethanol, Hydrobromierung der erhaltenen Doppelbindung und abschließender Grignard-Reaktion mit Isopropylmagnesiumbromid konnte eine racemische Mischung aus cis- und trans-Dihydrorosenoxid erhalten werden.

Liu et al. beschreiben in J. Heterocyclic Chem, 21, 129-132 (1984) die Herstellung von cis-Dihydrorosenoxid durch Hydrierung von 2-Isobutyl-4-methyl-5,6-dihydro-4H-pyran mit PtO₂ in Essigsäure.

Schindler und Vogel beschreiben in Perfume & Flavorist, Vol 11, 29-30 (1986) schematisch die Herstellung von Dihydrorosenoxid aus 3-Methylbut-3-en-1-ol und 3-Methylbutanal als Ausgangsmaterial, wobei eine cis/trans-Mischung im Verhältnis 70 : 30 erhalten wird. Weder der Reaktionsweg noch die einzuhaltenden Bedingungen sind genauer beschrieben.

Die EP 0 770 670 B1 beschreibt eine Parfümzusammensetzung, die 2-substituierte (4R)-cis-4-Methyl-tetrahydro-2H-pyrane enthält. In der Anmeldung werden die Geruchseigenschaften der Isomeren von Rosenoxid und Dihydrorosenoxid beschrieben. Die Synthese der Isomeren des Dihydrorosenoxids erfolgt durch Hydrierung der entsprechenden Isomeren des Rosenoxids.

Es besteht weiterhin ein großer Bedarf an effektiven Verfahren zur Herstellung von 2-substituierten 4-Methyl-tetrahydropyranen aus leicht verfügbaren Ausgangsstoffen. Neben der Synthese aus Reinsubstanzen ist dabei speziell auch die Nutzung von bisher nicht verwertbaren Nebenprodukten aus anderen Syntheseverfahren von Interesse. Dazu zählt insbesondere die integrierte Herstellung von wenigstens zwei Aroma- oder Geschmacksstoffen, ausgehend von einer Grundreaktion.

2-substituierte 4-Hydroxy-4-methyl-tetrahydropyrane sind ebenfalls wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetra-hydropyrans 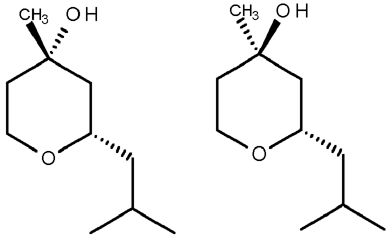 durch einen angenehmen Maiglöckchenduft aus und ist in besonderem Maße zur Verwendung als Aromachemikalie, z. B. zur Herstellung von Riechstoffkompositionen, geeignet.

Die EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d. h. der Aldehyd im Überschuss eingesetzt wird. Darüber hinaus offenbart das Dokument die anschließende Dehydratisierung der genannten Gemische zu den gewünschten ethylenisch ungesättigten Pyranen.

Die WO 2011/147919 beschreibt ein Verfahren zur Herstellung von 2-substituierten 4-Hydroxy-4-methyl-tetrahydropyranolen und speziell von 2-Isobutyl-4-hydroxy-4-methyl-tetrahydropyran durch Umsetzung von Isoprenol mit Prenal und anschließender Hydrierung.

Die WO 2010/133473 beschreibt ein Verfahren zur Herstellung von 2-substituierten 4-Hydroxy-4-methyl-tetrahydropyranen der Formel (I) 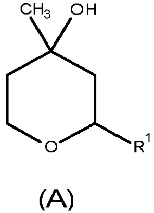 wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen steht, bei dem man Isoprenol (3-Methylbut-3-en-1-ol) mit einem Aldehyd der Formel R¹-CHO umsetzt, wobei man die Umsetzung in Gegenwart von Wasser und in Gegenwart eines stark sauren Kationenaustauschers durchführt.

Die WO 2011/154330 beschreibt ein zur WO 2010/133473 vergleichbares Verfahren, wobei das erhaltene Reaktionsgemisch einer destillativen Aufarbeitung in einer Trennwandkolonne oder in zwei thermisch gekoppelten Destillationskolonnen durchgeführt wird.

Wie in der WO 2010/133473 und WO 2011/154330 beschrieben ist, fällt bei der sauer katalysierten Umsetzung von Isoprenol (3-Methylbut-3-en-1-ol) mit einem Aldehyd der Formel R¹-CHO ein komplexes Reaktionsgemisch an, das neben 2-substituierten 4-Hydroxy-4-methyltetrahydropyranen noch dehydratisierte Nebenprodukte der Formeln (D), (E) und/oder (F) 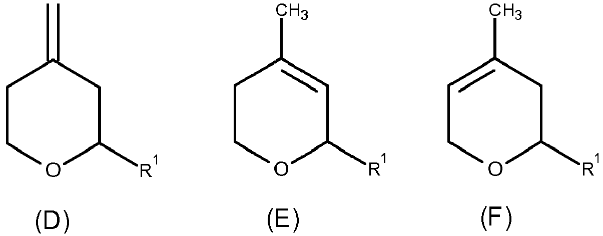 sowie als weitere Nebenprodukte unter anderem die 1,3-Dioxane (G) 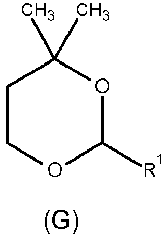 enthält. Diese Nebenprodukte konnten bis vor kurzem nicht zur Bereitstellung weiterer Wertstoffe genutzt werden, sondern wurden entweder ausgeschleust oder gemeinsam mit den im Überschuss eingesetzten Ausgangsverbindungen wieder in die Umsetzung von Isoprenol mit dem Aldehyd zurückgeführt. Letzteres ist aufgrund einer möglichen Aufpegelung dieser Komponenten im Reaktionsgemisch nicht unproblematisch.

Die unveröffentlichte internationale Patentanmeldung PCT/EP2013/071409 beschreibt ein Verfahren zur Herstellung von 2-substituierten 4-Hydroxy-4-methyl-tetrahydropyranen der allgemeinen Formel (I) und von 2-substituierten 4-Methyl-tetrahydropyranen der allgemeinen Formel (II) 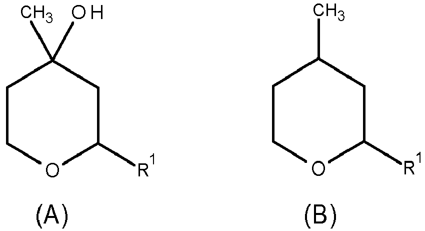 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol mit einem Aldehyd der Formel R¹-CHO, wobei R¹ in der Formel die zuvor angegebene Bedeutung hat, in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (A), wenigstens eine der Verbindungen (D), (E) oder (F) und wenigstens eine Dioxanverbindung (G) enthält 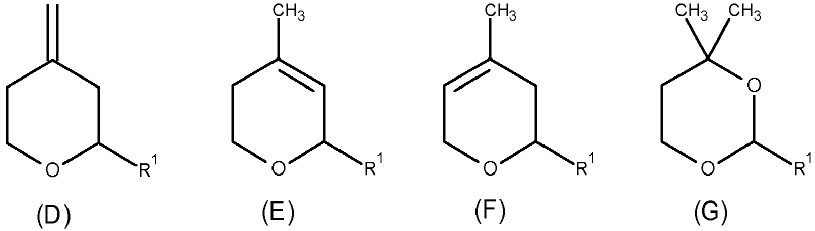 wobei R¹ die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (A) angereicherten Fraktion und einer Fraktion, die wenigstens eine der Verbindungen (D), (E) oder (F) und wenigstens eine Dioxanverbindung (G) enthält, unterzieht,
c) die Fraktion, die wenigstens eine der Verbindungen (D), (E) oder (F) und wenigstens eine Dioxanverbindung (G) enthält, einer Hydrierung unterzieht,
d) aus dem in Schritt c) erhaltenen Hydrierprodukt eine an 2-substituierten 4-Methyl-tetrahydropyranen (B) angereicherte Fraktion und eine an der wenigstens einen Dioxanverbindung (G) angereicherte Fraktion isoliert.

Romanov et al. beschreiben im Journal of Applied Chem. of the USSR, 55 (1), S. 140 - 143 (1982) (englische Übersetzung aus Zhurnal Prikladnoi Khimii, Bd. 55, Nr. 1, 157 - 161 (1981)) die sauer katalysierte Umsetzung der Dioxanverbindung G') zu den Dihydropyranen E') und F').

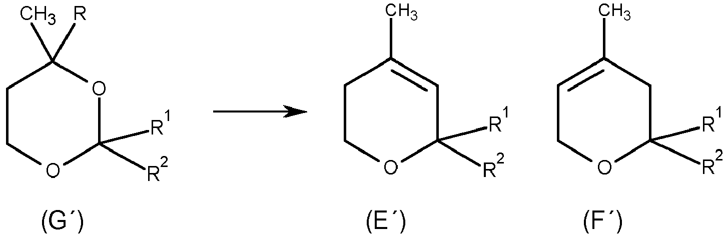

Tabellarisch erwähnt sind 4-Methyl-2-isobutyl-5,6-dihydropyran und 4-Methyl-2-isobutyl-3,6-dihydropyran. Als saure Katalysatoren werden H₂SO₄ oder Sulfonsäuregruppen-haltige Styrol-Divinylbenzol-Ionenaustauscher eingesetzt. Die Reaktion erfolgt mit Dioxanverbindungen G') in Reinform und in Gegenwart von Cyclohexan oder Toluol als Lösungsmittel.

Romanov et al. beschreiben im Journal of Applied Chem. of the USSR, 56 (1), S. 2526 - 2528 (1983) (englische Übersetzung aus Zhurnal Prikladnoi Khimii, Bd. 55, Nr. 12, 2778 - 2780 (1982)) die sauer katalysierte Isomerisierung von 2-R-4,4-Dimethyl- und 2-R-4-Methyl-4-phenyl-1,3-dioxanen zu 2-R-4,4-Methyl- und 2-R-4-Phenyl-1,3-tetrahydropyran-4-olen.

Der vorliegenden Erfindung liegt die Aufgabe zugrunde, ein verbessertes Verfahren zur Herstellung von 2-substituierten 4-Methyl-tetrahydropyranen zur Verfügung zu stellen.

Überraschenderweise wurde jetzt gefunden, dass durch Umsetzung von 2-Alkyl-4,4-dimethyl-1,3-dioxan-haltigen Ausgangsstoffen in Gegenwart einer starken Säure und/oder eines sauren Ionenaustauschers Produktgemische erhalten werden, die an wenigstens einem von drei isomeren Dihydropyranen, im Folgenden durch die Formeln (III.1), (III.2) und (III.3) charakterisiert, angereichert sind. Diese Produktgemische können durch Hydrierung in 2-substituierte 4-Methyl-tetrahydropyrane und speziell in Dihydrorosenoxid überführt werden.

Überraschenderweise wurde weiterhin gefunden, dass durch saure Umsetzung eines Ausgangsgemisches, das neben dem 2-Alkyl-4,4-dimethyl-1,3-dioxan bereits wenigstens ein solches Dihydropyran der Formeln (III.1), (III.2) und (III.3) enthält, der Gehalt des Produktgemisches an diesen Dihydropyranen weiter gesteigert werden kann.

Weiterhin wurde überraschenderweise gefunden, dass durch saure Umsetzung eines Ausgangsgemisches, das neben dem 2-Alkyl-4,4-dimethyl-1,3-dioxan wenigstens ein Dihydropyran (III.1), (III.2) und (III.3) und zusätzlich wenigstens ein 2-substituiertes 4-Hydroxy-4-methyl-tetrahydropyran (im Folgenden durch die Formel (VI) charakterisiert) enthält, der Gehalt des Produktgemisches sowohl an den Dihydropyranen der Formeln (III.1), (III.2) und/oder (III.3) als auch an dem 2-substituierten 4-Hydroxy-4-methyl-tetrahydropyran (VI) weiter gesteigert werden kann. Die erfindungsgemäße Säurebehandlung eignet sich somit für ein Verfahren zur integrierten Herstellung von 2-substituierten 4-Hydroxy-4-methyl-tetrahydropyranen und von 2-substituierten 4-Methyl-tetrahydropyranen.

Speziell wurde gefunden, dass sich der bei der sauer katalysierten Herstellung von 2-substituierten 4-Hydroxy-4-methyl-tetrahydropyranen durch Umsetzung von Isoprenol (3-Methylbut-3-en-1-ol) mit einem geeigneten Aldehyd (speziell Isovaleraldehyd) anfallende Dioxan-haltige Seitenstrom (= Abfallstrom) durch die erfindungsgemäße Säurebehandlung zu einem Großteil der Verwendung als Aromachemikalie und speziell als Duftstoff zugeführt werden kann.

### ZUSAMMENFASSUNG DER ERFINDUNG

Gegenstand der Erfindung ist ein Verfahren zur Herstellung von 2-substituierten 4-Methyl-tetrahydropyranen der allgemeinen Formel (I) 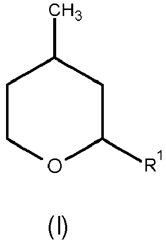 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) ein Ausgangsmaterial bereitstellt, das wenigstens eine Dioxanverbindung der allgemeinen Formel (II) enthält 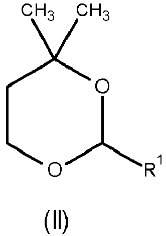 wobei R¹ die zuvor angegebene Bedeutung hat,
b) das Ausgangsmaterial einer Umsetzung in Gegenwart einer starken Säure und/oder eines sauren Ionenaustauscher unterzieht, wobei ein gegenüber dem Ausgangsmaterial an der Dioxanverbindung der Formel (II) abgereichertes und an wenigstens einer der Verbindungen der Formeln (III.1), (III.2) oder (III.3) 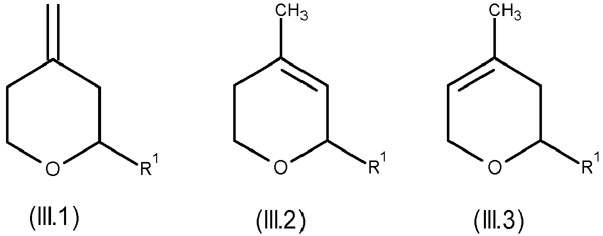 angereichertes Produktgemisch erhalten wird, wobei R¹ die zuvor angegebene Bedeutung hat,
c) das in Schritt b) erhaltene Produktgemisch einer Hydrierung unterzieht.

### BESCHREIBUNG DER ERFINDUNG

Das erfindungsgemäße Verfahren weist die folgenden Vorteile auf:
- Mit dem erfindungsgemäßen Verfahren lassen sich 2-Alkyl-4,4-dimethyl-1,3-dioxan-haltige Ausgangsstoffe zur Herstellung von 2-substituierten 4-Methyl-tetrahydropyranen nutzen, d. h. der Verwendung als Aromachemikalie und speziell als Duftstoff zuführen.
- Die nach der erfindungsgemäßen Säurebehandlung erhaltenen Produktgemische zeichnen sich durch ihre geringen Restgehalte an 2-Alkyl-4,4-dimethyl-1,3-dioxanen aus. Die aus dem Stand der Technik bekannten Probleme bei der Auftrennung und/oder Weiterverarbeitung solcher Dioxan-haltiger Ströme werden vermieden bzw. deutlich verringert.

- Mit dem erfindungsgemäßen Verfahren kann speziell ein Großteil des bisherigen Seitenstroms (Abfallstroms) bei der sauer katalysierten Herstellung von 2-substituierten 4-Hydroxy-4-methyl-tetrahydropyranen als Wertstoff verwendet werden. Aufgrund der zuvor beschriebenen Probleme bei der Trennung der in diesem Seitenstrom enthaltenen Dihydropyrane der Formeln (III.1), (III.2) und/oder (III.3) einerseits und des 2-Alkyl-4,4-dimethyl-1,3-dioxans (II) andererseits, wurde dieser Seitenstrom bislang in der Regel der Verbrennung zugeführt.
- Mit der erfindungsgemäß vorgesehenen Hydrierung wird ein Zugang zu 2-substituierten 4-Methyl-tetrahydropyranen und speziell zu Dihydrorosenoxid ermöglicht, der nur zwei Reaktionsstufen, ausgehend vom Seitenstrom, erfordert.
- Zur Herstellung der 2-substituierten 4-Methyl-tetrahydropyrane, speziell des Dihydrorosenoxids, müssen keine weiteren teuren und/oder potentiell gefährlichen Reagenzien, wie etwa Grignard-Reagenzien oder komplexe Hydride, wie Lithiumaluminiumhydrid, eingesetzt werden.

Sofern im Folgenden nicht genauer angegeben, bezeichnen die Begriffe
"2-substituiertes 4-Methyl-tetrahydropyran",
"2-(2-Methylpropyl)-4-methyl-tetrahydropyran" (= "Dihydrorosenoxid"),
"2-substituiertes 4-Hydroxy-4-methyl-tetrahydropyran",
"2-(2-Methylpropyl)-4-hydroxy-4-methyl-tetrahydropyran"

im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I) oder (II) die Rede ist, wird jeweils nur eine der enantiomeren Formen abgebildet. Lediglich zur Veranschaulichung werden im Folgenden die Isomeren des 2-(2-Methylpropyl)-4-methyl-tetrahydropyrans (I) (Dihydrorosenoxid) wiedergegeben:

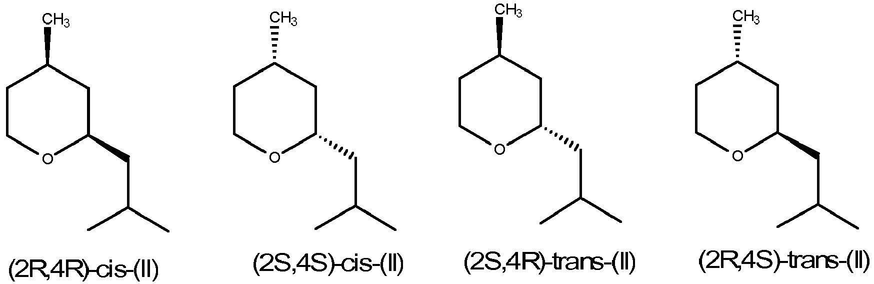

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy.

Im Rahmen der vorliegenden Erfindung steht der Ausdruck geradkettiges oder verzweigtes Alkenyl vorzugsweise für C₂-C₆-Alkenyl und besonders bevorzugt für C₂-C₄-Alkenyl. Der Alkenylrest weist neben Einfachbindungen noch eine oder mehrere, bevorzugt 1 bis 3, besonders bevorzugt 1 oder 2 und ganz besonders bevorzugt eine ethylenische Doppelbindung auf. Alkenyl steht insbesondere für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl oder 2-Methyl-2-propenyl.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3-und 4-Propylcyclohexyl, 2-, 3- und 4-Isopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc. und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

Bevorzugt steht R¹ in den Verbindungen der Formel (I), (II), (III.1), (III.2), (III.3), (V), und (VI) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Die vorliegende Erfindung betrifft somit im Rahmen einer bevorzugten Ausführungsform ein Verfahren zur Herstellung und Isolierung von 2-(2-Methylpropyl)-4-methyl-tetrahydropyran der Formel (la) (Dihydrorosenoxid).

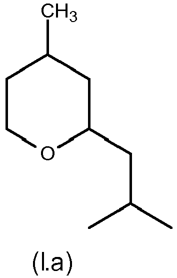

### Schritt a)

Geeignete Ausgangsmaterialien für den Einsatz in Schritt a) können wenigstens eine Dioxanverbindung der allgemeinen Formel (II) 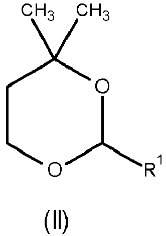 wobei R¹ die zuvor angegebene Bedeutung hat, in im Wesentlichen reiner Form enthalten. Unter im Wesentlichen reiner Form wird verstanden, dass das für den Einsatz in Schritt a) eingesetzte Ausgangsmaterial vorzugsweise zu wenigstens 90 Gew.-%, besonders bevorzugt zu wenigstens 95 Gew.-%, insbesondere zu wenigstens 99 Gew.-%, bezogen auf das Gesamtgewicht des Ausgangsmaterials, aus wenigstens einer Dioxanverbindung der allgemeinen Formel (II) besteht.

Geeignete Verfahren zur Herstellung von 1,3-Dioxanen der Formel (II) sind dem Fachmann prinzipiell bekannt. Dazu zählt beispielsweise die Umsetzung von 3-Methyl-1,3-butandiol mit entsprechend substituierten Aldehyden nach folgendem Schema: 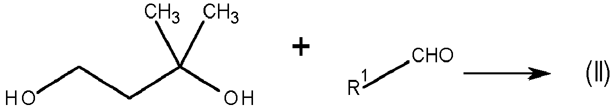 3-Methyl-1,3-butanediol ist kommerziell erhältlich, z. B. von Sigma Aldrich. Das Gleiche gilt für viele Aldehyde der Formel R¹-CHO.

Bevorzugt wird in Schritt a) ein Ausgangsmaterial bereitgestellt, das zusätzlich zu wenigstens einer Dioxanverbindung (II) wenigstens eine weitere Komponente enthält.

Bevorzugt handelt es sich bei dem in Schritt a) bereitgestellten Ausgangsmaterial um ein natürliches oder synthetisches Produktgemisch der Synthese und/oder der Isolierung eines von 2-substituierten 4-Methyl-tetrahydropyranen der allgemeinen Formel (I) verschiedenen Aroma- oder Geschmacksstoffs.

Bevorzugt enthält das in Schritt a) bereitgestellte Ausgangsmaterial die wenigstens eine Dioxanverbindung (II) in einer Menge von 1 bis 99 Gew.-%, bevorzugt von 2 bis 90 Gew.-%, insbesondere von 5 bis 50 Gew.-%, speziell 15 bis 50 Gew.-%, bezogen auf das Gesamtgewicht des Ausgangsmaterials.

In einer bevorzugten Ausführungsform wird in Schritt a) ein Ausgangsmaterial bereitgestellt, das zusätzlich zu wenigstens einer Dioxanverbindung (II) wenigstens eine der Verbindungen der Formeln (III.1), (III.2) oder (III.3) 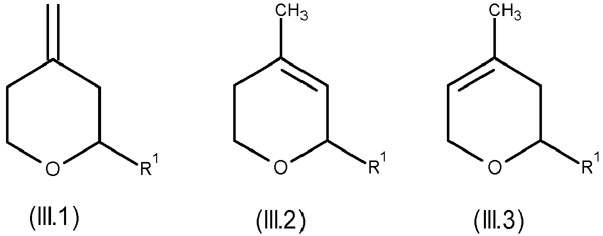 enthält, worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

Produktgemische, die wenigstens eine der Dihydropyran-Verbindungen der Formeln (III.1), (III.2) oder (III.3) enthalten, können, wie zuvor ausgeführt, durch Hydrierung in 2-substituierte 4-Methyl-tetrahydropyrane und speziell in Dihydrorosenoxid überführt werden. Vorteilhafterweise kann durch Umsetzung eines Ausgangsmaterials, das wenigstens eine Dioxanverbindung (II) und wenigstens eine der Verbindungen der Formeln (III.1), (III.2) oder (III.3) enthält, in Gegenwart einer starken Säure und/oder eines sauren Ionenaustauschers, der Gehalt des erhaltenen Produktgemisches an Verbindungen der Formeln (III.1), (III.2) und (III.3) gegenüber dem Ausgangsmaterial weiter gesteigert werden. Gleichzeitig wird ein gegenüber dem Ausgangsmaterial an der Dioxanverbindung der Formel (II) abgereichertes Produktgemisch erhalten.

Bevorzugt enthält das in Schritt a) bereitgestellte Ausgangsmaterial die Verbindungen der Formeln (III.1), (III.2) und/oder (III.3) in einer Gesamtmenge von 1 bis 99 Gew.-%, bevorzugt von 5 bis 95 Gew.-%, insbesondere von 15 bis 70 Gew.-%, bezogen auf das Gesamtgewicht des Ausgangsmaterials.

In einer typischen Zusammensetzung enthält das in Schritt a) bereitgestellte Ausgangsmaterial die folgenden Verbindungen, jeweils bezogen auf das Gesamtgewicht des Reaktionsgemischs:
Isovaleraldehyd: 0 - 15 Gew.-%,
Isoprenol: 0 - 15 Gew.-%,
Dioxanverbindung (II): 15 - 50 Gew.-%,
Verbindungen der Formeln (III.1), (III.2) und/oder (III.3): insgesamt 15 - 70 Gew.-%, von (II) verschiedene Acetale: 0 - 5 Gew.-%,
2-substituierte 4-Hydroxy-4-methyl-tetrahydropyrane (VI): 0 - 30 Gew.-%,
Wasser: 0 - 10 Gew.-%.

Eine spezielle Ausführungsform ist ein Verfahren, wobei man zur Bereitstellung des Ausgangsmaterials in Schritt a):
a1) 3-Methylbut-3-en-1-ol der Formel (IV) 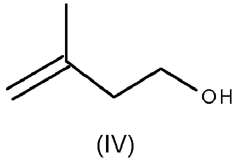 mit einem Aldehyd der Formel (V)

   R¹-CHO (V)

   worin
   - R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
   in Gegenwart eines sauren Katalysators umsetzt, wobei ein Reaktionsgemisch erhalten wird, das wenigstens eine Dioxanverbindung (II), wenigstens eine der Verbindungen (III.1), (III.2) oder (III.3) und wenigstens ein 2-substituiertes 4-Hydroxy-4-methyl-tetrahydropyran der allgemeinen Formel (VI) 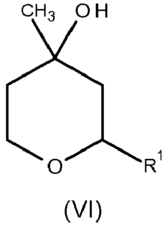 enthält, wobei R¹ in der Formel (VI) die zuvor angegebene Bedeutung hat,
a2) gegebenenfalls das Reaktionsgemisch aus Schritt a1) einer Auftrennung unter Erhalt wenigstens einer an 2-substituierten 4-Hydroxy-4-methyl-tetrahydropyranen der allgemeinen Formel (VI) angereicherten Fraktion und einer Fraktion, die die Dioxanverbindung (II) und wenigstens eine der Verbindungen (III.1), (III.2) oder (III.3) enthält, unterzieht,
   und das in Schritt a1) erhaltene Reaktionsprodukt oder die in Schritt a2) erhaltene Fraktion, die die Dioxanverbindung (II) und wenigstens eine der Verbindungen (III.1), (III.2) oder (III.3) enthält, als Ausgangsmaterial zur Herstellung der 2-substituierten 4-Methyl-tetrahydropyrane der Formel (I) einsetzt.

Speziell bevorzugt ist ein Verfahren, wobei man zur Bereitstellung des Ausgangsmaterials in Schritt a):
a1) 3-Methylbut-3-en-1-ol der Formel (IV) 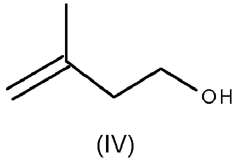 mit einem Aldehyd der Formel (V)

   R¹-CHO (V)

   worin
   - R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
   in Gegenwart eines sauren Katalysators umsetzt, wobei ein Reaktionsgemisch erhalten wird, das wenigstens eine Dioxanverbindung (II), wenigstens eine der Verbindungen (III.1), (III.2) oder (III.3) und wenigstens ein 2-substituiertes 4-Hydroxy-4-methyl-tetrahydropyran der allgemeinen Formel (VI) 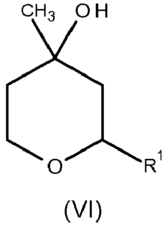 enthält, wobei R¹ in der Formel (VI) die zuvor angegebene Bedeutung hat,
a2) das Reaktionsgemisch aus Schritt a1) einer Auftrennung unter Erhalt wenigstens einer an 2-substituierten 4-Hydroxy-4-methyl-tetrahydropyranen der allgemeinen Formel (VI) angereicherten Fraktion und einer Fraktion, die die Dioxanverbindung (II) und wenigstens eine der Verbindungen (III.1), (III.2) oder (III.3) enthält, unterzieht,
   und die in Schritt a2) erhaltene Fraktion, die die Dioxanverbindung (II) und wenigstens eine der Verbindungen (III.1), (III.2) oder (III.3) enthält, als Ausgangsmaterial zur Herstellung der 2-substituierten 4-Methyl-tetrahydropyrane der Formel (I) einsetzt.
   Vorteilhafterweise ermöglicht die zuvor genannte speziell bevorzugte Ausführungsform ein integriertes Verfahren zur gleichzeitigen Herstellung von 2-substituierten 4-Methyl-tetrahydropyranen und von 2-substituierten 4-Hydroxy-4-methyl-tetrahydropyranen.

Die vorliegende Erfindung betrifft somit im Rahmen einer speziell bevorzugten Ausführungsform ein Verfahren zur Herstellung und Isolierung von 2-(2-Methylpropyl)-4-methyl-tetrahydropyran der Formel (la) (Dihydrorosenoxid) und von 2-(2-Methylpropyl)-4-hydroxy-4-methyl-tetrahydropyran der Formel (IIa):

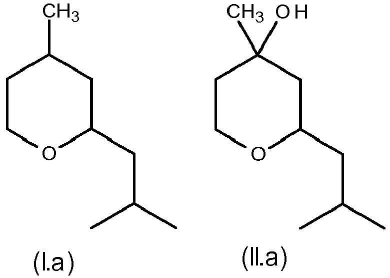

### Schritt a1)

Einer der Ausgangsstoffe für Schritt a1) des erfindungsgemäßen Verfahrens ist 3-Methylbut-3-en-1-ol (Isoprenol) der Formel (IV),

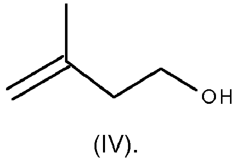

Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in Schritt a) des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für Schritt a1) des erfindungsgemäßen Verfahrens ist ein Aldehyd der Formel (V) R¹-CHO, wobei R¹ in der Formel (V) die zuvor angegebene Bedeutung hat.

Bevorzugt einzusetzende Aldehyde der Formel (V) sind: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (V) sind Isovaleraldehyd und Benzaldehyd, insbesondere Isovaleraldehyd.

Bevorzugt werden in Schritt a1) das 3-Methylbut-3-en-ol (IV) und der Aldehyd (V) in einem molaren Verhältnis von etwa 1 zu 2 bis 2 zu 1, besonders bevorzugt von 0,7 zu 1 bis 2 zu 1, insbesondere von 1 zu 1 bis 2 zu 1, eingesetzt. In einer speziellen Ausführung werden in Schritt a) das 3-Methylbut-3-en-ol (IV) und der Aldehyd (V) in einem molaren Verhältnis von 1 zu 1 bis 1,5 zu 1 eingesetzt.

Bevorzugt erfolgt die Umsetzung in Schritt a1) in Gegenwart eines sauren Katalysators. Prinzipiell kann für die Umsetzung in Schritt a1) jeder saure Katalysator verwendet werden, d. h. jede Substanz, die Brönstedt- oder Lewis-Acidität aufweist. Beispiele für geeignete Katalysatoren sind Protonensäuren, wie Salzsäure, Schwefelsäure, Phosphorsäure, Methansulfonsäure und p-Toluolsulfonsäure, saure molekulare Elementverbindungen, wie Aluminiumchlorid, Bortrifluorid, Zinkchlorid, Phosphorpentafluorid, Arsentrifluorid, Zinntetrachlorid, Titantetrachlorid und Antimonpentafluorid; oxidische saure Festkörper wie Zeolithe, Silikate, Aluminate, Alumosilikate, Tone und saure Ionentauscher.

Bevorzugt ist der in Schritt a1) eingesetzte saure Katalysator ausgewählt unter Salzsäure, Schwefelsäure, Methansulfonsäure, p-Toluolsulfonsäure und stark sauren Kationenaustauschern.

In einer ersten Variante erfolgt die Umsetzung in Schritt a1) in Gegenwart einer Brönstedt-Säure, die vorzugsweise ausgewählt ist unter Salzsäure, Schwefelsäure, Methansulfonsäure, p-Toluolsulfonsäure. In dieser ersten Variante kann in Schritt a1) ein Lösungsmittel eingesetzt werden, das vorzugsweise ausgewählt ist unter Kohlenwasserstoffen und Kohlenwasserstoffgemischen. Geeignete Lösungsmittel sind beispielsweise Hexan, Heptan, Ligroin, Petrolether, Cyclohexan, Dekalin, Toluol, Xylol und Mischungen davon. Bevorzugt wird kein Lösungsmittel eingesetzt. Bevorzugt wird der Katalysator in dieser ersten Variante in einer Menge von 0,05 bis 5 Mol-%, besonders bevorzugt von 0,1 bis 4 Mol-%, bezogen auf den Aldehyd (V), eingesetzt. Bevorzugt erfolgt die Umsetzung in Schritt a1) nach dieser ersten Variante bei einer Temperatur im Bereich von 20 bis 120 °C, besonders bevorzugt 30 bis 80 °C.

In einer zweiten Variante erfolgt die Umsetzung in Schritt a1) in Gegenwart eines stark sauren Kationenaustauschers. Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben, worauf hier in vollem Umfang Bezug genommen wird.

Geeignet für den Einsatz in Schritt a1) sind stark saure Ionenaustauscher (wie z. B. Amberlyst, Amberlite, Dowex, Lewatit, Purolite, Serdolit), die auf Polystyrol basieren und die Copolymere aus Styrol und Divinylbenzol als Trägermatrix mit Sulfonsäuregruppen in H⁺-Form enthalten sowie mit Sulfonsäuregruppen (-SO₃H) funktionalisierte Ionenaustauschergruppen. Die Ionenaustauscher unterscheiden sich im Aufbau ihrer Polymergerüste, und man unterscheidet gelförmige und makroporöse Harze. In einer speziellen Ausführung wird in Schritt a) ein perfluoriertes polymeres lonenaustauscherharz eingesetzt. Derartige Harze werden z. B. unter der Bezeichnung Nafion ® von der Firma DuPont vertrieben. Als Beispiel für ein solches perfluoriertes polymeres lonenaustauscherharz sei Nafion ® NR-50 genannt.

Für die Umsetzung in Schritt a1) geeignete kommerziell verfügbare stark saure Kationenaustauscher sind beispielsweise unter den Handelsnamen Lewatit ® (Lanxess), Purolite ® (The Purolite Company), Dowex ® (Dow Chemical Company), Amberlite ® (Rohm and Haas Company), Amberlyst (TM) (Rohm and Haas Company) bekannt. Bevorzugte stark saure Kationenaustauscher sind: Lewatit ® K 1221, Lewatit ® K 1461, Lewatit ® K 2431, Lewatit ® K 2620, Lewatit ® K 2621, Lewatit ® K 2629, Lewatit ® K 2649, Amberlite ® FPC 22, Amberlite ® FPC 23, Amberlite ® IR 120, Amberlyst (TM) 131, Amberlyst (TM) 15, Amberlyst (TM) 31, Amberlyst (TM) 35, Amberlyst (TM) 36, Amberlyst (TM) 39, Amberlyst (TM) 46, Amberlyst (TM) 70, Purolite ® SGC650, Purolite ® C100H, Purolite ® C150H, Dowex ® 50X8, Serdolit ® rot und Nation ® NR-50.

Die stark sauren lonentauscherharze werden in der Regel mit Salzsäure und/oder Schwefelsäure regeneriert.

In einer speziellen Ausführung werden in Schritt a1) das 3-Methylbut-3-en-ol (IV) und der Aldehyd (V) in Gegenwart eines stark sauren Kationenaustauschers und in Gegenwart von Wasser umgesetzt. Nach einer speziellen Ausführung wird dem Reaktionsgemisch neben Isoprenol (IV) und dem Aldehyd der Formel (V) zusätzlich noch Wasser zugesetzt.

In einer geeigneten Ausgestaltung werden die Ausgangsstoffe in Gegenwart von mindestens 25 Mol-%, bevorzugt von mindestens 50 Mol-% Wasser umgesetzt. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

Vorzugsweise führt man die Umsetzung in Gegenwart von etwa mindestens 3 Gew.-%, besonders bevorzugt von mindestens 5 Gew.-% zugesetztem Wasser durch. Der Alkohol der Formel (IV) und der Aldehyd der Formel (V) werden beispielsweise in Gegenwart von 3 Gew.-% bis 15 Gew.-% Wasser, bevorzugt von 5 Gew.-% bis 12 Gew.-% Wasser umgesetzt. Die angegebenen vorstehenden Gew.-% sind dabei bezogen auf die Gesamtmenge des Reaktionsgemisches, bestehend aus den Komponenten der Formeln (IV) und (V) sowie Wasser.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 5- bis 15-fachem Überschuss oder auch darüber eingesetzt werden. Vorzugsweise bereitet man ein Gemisch aus Isoprenol (IV) und dem Aldehyd der Formel (V), vorzugsweise Isovaleraldehyd, mit der zuzusetzenden Menge Wasser, so dass das zugegebene Wasser in dem Gemisch aus Isoprenol und dem Aldehyd gelöst bleibt, d. h. kein zweiphasiges System vorliegt.

Zur Umsetzung von Isoprenol (IV) mit dem Aldehyd (V) in Schritt a1) kann man die genannten Ausgangsstoffe und gegebenenfalls das zugesetzte Wasser mit dem sauren Kationenaustauscher in Kontakt bringen. Vorzugsweise werden Isoprenol (IV), Aldehyd (V) und gegebenenfalls das zugesetzte Wasser in Form eines Gemisches in Schritt a) eingesetzt. Die genannten Ausgangsstoffe, d. h. Isoprenol (IV) und der Aldehyd (V) und das in der vorstehenden Menge einzusetzende Wasser können in beliebiger Reihenfolge miteinander in Kontakt gebracht bzw. gemischt werden.

Die Menge an stark saurem Kationenaustauscher in Schritt a1) ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Üblicherweise setzt man den stark sauren Kationentauscher in einer Menge von etwa 5 bis etwa 40 Gew.-%, bevorzugt in einer Menge von etwa 20 bis etwa 40 Gew.- % und besonders bevorzugt in einer Menge von etwa 20 bis etwa 30 Gew.-%, jeweils bezogen auf die Summe an eingesetztem Isoprenol (IV) und Aldehyd der Formel (V), ein. Dabei beziehen sich die Angaben auf den gebrauchsfertigen Kationenaustauscher, der in der Regel mit Wasser vorbehandelt wird und dementsprechend Mengen von bis zu etwa 70 Gew.-%, bevorzugt von etwa 30 bis etwa 65 Gew.-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen. Die genannten stark sauren Kationenaustauscher können in Schritt a1) sowohl einzeln oder auch in Form von Gemischen eingesetzt werden.

Bei kontinuierlicher Fahrweise liegt die Katalysatorbelastung beispielsweise im Bereich von 50 bis 2500 mol pro m³ Katalysator und h, bevorzugt im Bereich von 100 bis 2000 mol pro m³ Katalysator und h, insbesondere im Bereich von 130 bis 1700 mol pro m³ Katalysator und h, wobei sich die Stoffmenge in mol auf den Ausgangsstoff der Formel (IV) bezieht.

Die Umsetzung in Schritt a1) in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a1) in Gegenwart eines stark sauren Kationenaustauschers ohne Zusatz eines organischen Lösungsmittels durch.

Bevorzugt wird die Umsetzung von Isoprenol (IV) mit dem gewählten Aldehyd (V) in Schritt a1) in Gegenwart von Wasser und in Gegenwart eines stark sauren Kationentauschers bei einer Temperatur im Bereich von 0 bis 70 °C, besonders bevorzugt bei einer Temperatur im Bereich von 20 bis 70 °C und insbesondere bei einer Temperatur im Bereich von 20 bis 60 °C durchgeführt. Dabei handelt es sich um die Temperatur des Reaktionsgemisches.

Die Umsetzung in Schritt a1) kann diskontinuierlich oder kontinuierlich durchgeführt werden. Dabei kann man beispielsweise im diskontinuierlichen Fall die Umsetzung so vornehmen, dass man ein Gemisch von Isoprenol (IV), dem Aldehyd (V), gegebenenfalls Wasser und gegebenenfalls einem organischen Lösungsmittel in einem geeigneten Reaktionsgefäß vorlegt und den sauren Katalysator zugibt. Nach Abschluss der Reaktion kann dann der Katalysator durch geeignete Trennverfahren vom erhaltenen Reaktionsgemisch abgetrennt werden. Die Reihenfolge des Inkontaktbringens der einzelnen Reaktionskomponenten ist nicht kritisch und kann nach Maßgabe der jeweiligen verfahrenstechnischen Ausgestaltung variiert werden. Wird in Schritt a1) eine Brönstedt-Säure, die vorzugsweise ausgewählt ist unter Salzsäure, Schwefelsäure, Methansulfonsäure, p-Toluolsulfonsäure, als Katalysator eingesetzt, so kann die Abtrennung des Katalysators, z. B. nach wässriger Aufarbeitung, destillativ erfolgen oder direkt destillativ erfolgen. Wird in Schritt a1) ein stark saurer Kationenaustauscher als Katalysator eingesetzt, so kann die Abtrennung des Katalysators z. B. durch Filtration oder durch Zentrifugation erfolgen.

Im Rahmen einer bevorzugten Ausführungsform führt man die Umsetzung von Isoprenol (IV) mit dem Aldehyd (V) in Schritt a) kontinuierlich durch. Dazu kann beispielsweise eine Mischung der umzusetzenden Ausgangsstoffe Isoprenol und Aldehyd der Formel (IV) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor, eingebracht werden und die Ausgangsstoffe und das Wasser in diesen kontinuierlich eingetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden. Entsprechende Verfahren sind in den europäischen Patentanmeldungen 13165767.8 und 13165778.5 beschrieben.

Das in Schritt a1) des erfindungsgemäßen Verfahrens erhaltene Reaktionsgemisch enthält neben wenigstens einer Dioxanverbindung der allgemeinen Formel (II), 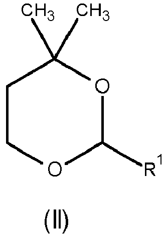 wenigstens eine der Verbindungen der Formeln (III.1), (III.2) oder (III.3) 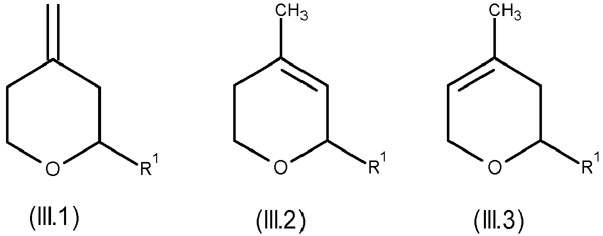 und wenigstens ein 2-substituiertes 4-Hydroxy-4-methyl-tetrahydropyran der allgemeinen Formel (VI) 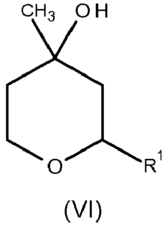 wobei R¹ in den Formeln (II), (III.1), (III.2), (III.3) und (VI) die zuvor angegebene Bedeutung hat. Bevorzugt steht R¹ für Isobutyl. In der Regel enthält das Reaktionsgemisch ein Gemisch der Verbindungen (III.1), (III.2) und (III.3).

Das in Schritt a1) des erfindungsgemäßen Verfahrens erhaltene Reaktionsgemisch kann wenigstens ein weiteres Nebenprodukt enthalten, z. B. ein Acetal (VII) 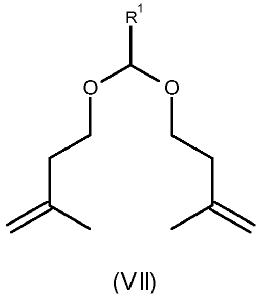 wobei R¹ die zuvor angegebene Bedeutung hat. Bevorzugt steht R¹ für Isobutyl.

Das in Schritt a1) des erfindungsgemäßen Verfahrens erhaltene Reaktionsgemisch kann weitere Komponenten enthalten, wie nicht umgesetztes 3-Methylbut-3-en-1-ol (IV), nicht umgesetzten Aldehyd (V), Wasser, organisches Lösungsmittel, etc.

Vorzugsweise enthält das in Schritt a1) erhaltene Reaktionsgemisch die Dioxan-Verbindung der Formel (II) in einer Gesamtmenge von 5 bis 20 Gew.-%, besonders bevorzugt 5 bis zu etwa 15 Gew.-%, bezogen auf das Gesamtgewicht des Reaktionsgemischs.

Vorzugsweise enthält das in Schritt a1) erhaltene Reaktionsgemisch die Verbindungen der Formeln (III.1), (III.2) und (III.3) in einer Gesamtmenge von 5 bis 20 Gew.-%, besonders bevorzugt 5 bis zu etwa 15 Gew.-%, bezogen auf das Gesamtgewicht des Reaktionsgemischs.

Vorzugsweise enthält das in Schritt a1) erhaltene Reaktionsgemisch das 2-substituierte 4-Hydroxy-4-methyl-tetrahydropyran der Formel (VI) in einer Menge von 50 bis 90 Gew.-%, besonders bevorzugt 60 bis zu etwa 80 Gew.-%, bezogen auf das Gesamtgewicht des Reaktionsgemischs.

In einer typischen Zusammensetzung enthält das in Schritt a1) erhaltene Reaktionsgemisch die folgenden Verbindungen, jeweils bezogen auf das Gesamtgewicht des Reaktionsgemischs:
Isovaleraldehyd: 0 - 5 Gew.-%,
Isoprenol: 0 - 10 Gew.-%,
Dioxanverbindung (II): 5 - 15 Gew.-%,
Verbindungen der Formeln (III.1), (III.2) und/oder (III.3): insgesamt 5 - 15 Gew.-%,
von (II) verschiedene Acetale: 0 - 5 Gew.-%,
trans-(VI): 15 - 22 Gew.-%,
cis-(VI): 45 - 65 Gew.-%,
Wasser: 2 - 10 Gew.-%.

Bevorzugt enthält das in Schritt a1) erhaltene Reaktionsgemisch die 2-substituierten 4-Hydroxy-4-methyl-tetrahydropyrane der Formel (VI) in Form von Gemischen der cis-Diastereomeren der Formel cis-(I) und der trans-Diastereomeren der Formel trans-(I) 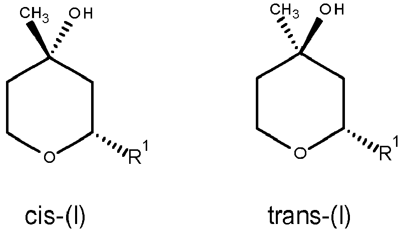 wobei das Diastereomerenverhältnis des cis-Diastereomeren cis-(VI) zum trans-Diastereomeren trans-(VI) bevorzugt 65 zu 35 bis 95 zu 5, besonders bevorzugt 70 zu 30 bis 85 zu 15 beträgt und R¹ die weiter oben angegebenen Bedeutungen hat.

Bevorzugt enthält das in Schritt a1) erhaltene Reaktionsgemisch 2-Isobutyl-4-hydroxy-4-methyl-tetrahydropyran in Form von Gemischen des cis-Diastereomeren der Formel cis-(VI.a) und des trans-Diastereomeren der Formel trans-(VI.a) 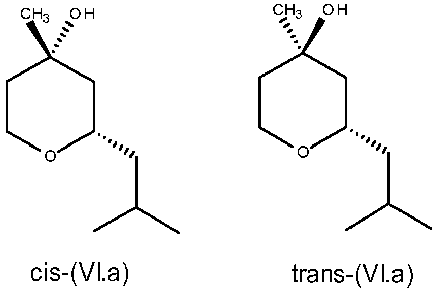 wobei das Diastereomerenverhältnis des cis-Diastereomeren cis-(VI.a) zum trans-Diastereomeren trans-(VI.a) bevorzugt 65 zu 35 bis 95 zu 5, besonders bevorzugt 70 zu 30 bis 85 zu 15 beträgt.

Derartige Gemische eignen sich aufgrund ihrer besonderen geruchlichen Eigenschaften in besonderem Masse zur Verwendung als Aromachemikalien, beispielsweise als Komponente mit Maiglöckchenduft zur Herstellung von Riechstoffkompositionen.

### Schritt a2)

Das in Schritt a1) erhaltene Reaktionsgemisch kann in einer ersten Ausführungsform ohne weitere Auftrennung zur Umsetzung in Schritt b) eingesetzt werden.

In einer zweiten bevorzugten Ausführungsform wird das in Schritt a1) erhaltene Reaktionsgemisch einer Auftrennung unter Erhalt wenigstens einer an 2-substituierten 4-Hydroxy-4-methyl-tetrahydropyranen der allgemeinen Formel (VI) angereicherten Fraktion und einer Fraktion, die die Dioxanverbindung (II) und wenigstens eine der Verbindungen (III.1), (III.2) oder (III.3) enthält, unterzogen. Die Fraktion, die die Dioxanverbindung (II) und wenigstens eine der Verbindungen (III.1), (III.2) oder (III.3) enthält, wird vorzugsweise als Ausgangsmaterial für die Umsetzung in Schritt b) eingesetzt.

Das zur Auftrennung in Schritt a2) eingesetzte Reaktionsprodukt aus Schritt a1) enthält, bezogen auf das Gesamtgewicht, typischerweise 45 bis 65 Gew.-% der cis-Diastereomeren cis-(VI), 15 bis 22 Gew.-% der trans-Diastereomeren trans-(VI), 10 bis 40 Gew.-% niedriger als die Verbindungen (VI) siedende Verbindungen, 1 bis 3 Gew.-% höher als die Verbindungen (VI) siedende Verbindungen. Das Reaktionsprodukt aus Schritt a1) ist vorzugsweise im Wesentlichen frei von Verbindungen, die einen Siedepunkt nahe dem der stereoisomeren Verbindungen (VI) aufweisen. Im Wesentlichen frei von Verbindungen, die einen Siedepunkt nahe dem der stereoisomeren Verbindungen (VI) aufweist, bedeutet im Rahmen der Erfindung, dass das Reaktionsprodukt aus Schritt a1) höchstens 1 Gew.-%, besonders bevorzugt höchstens 0,5 Gew.-%, insbesondere höchstens 0,1 Gew.-% an Verbindungen enthält, die einen Siedepunkt nahe dem der stereoisomeren Verbindungen (VI) aufweisen.

Bevorzugt enthält das zur Auftrennung in Schritt a2) eingesetzte Reaktionsprodukt aus Schritt a1) 45 bis 65 Gew.-% des cis-Diastereomeren von 2-Isobutyl-4-hydroxy-4-methyl-tetrahydropyran der Formel cis-(VI.a), 15 bis 20 Gew.-% des trans-Diastereomeren der Formel trans-(VI.a), 20 bis 40 Gew.-% niedriger als die Verbindungen (VI) siedende Verbindungen, 1 bis 3 Gew.-% höher als die Verbindungen (VI) siedende Verbindungen.

Bevorzugt wird in Schritt a2) des erfindungsgemäßen Verfahrens das Reaktionsgemisch aus Schritt a1) einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Besonders bewährt haben sich geordnete Packungen, vorzugsweise Blech- oder Gewebepackungen, mit einer spezifischen Oberfläche von 100 bis 750 m²/m³, insbesondere 250 bis 500 m²/m³. Sie gestalten hohe Trennleistungen bei niedrigen Druckverlusten.

Vorzugsweise wird zur Auftrennung in Schritt a2) eine Vorrichtung eingesetzt, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule
   umfasst.

Vorzugsweise erfolgt die Auftrennung in Schritt a2), indem man
i) das Reaktionsprodukt aus Schritt a1) in eine Zulaufsäule mit oberhalb der Zulaufstelle gelegenem Verstärkungsteil und unterhalb der Zulaufstelle gelegenem Abtriebsteil einführt,
ii) eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Aufheizer am unteren Säulenende vorsieht,
iii) eine mit der oberen Vereinigungssäule und der unteren Vereinigungssäule kommunizierende Abzugssäule vorsieht, die wenigstens einen Seitenabzug aufweist,
iv) aus der Abzugssäule am Kopf oder im oberen Bereich leichter als die 2-substituierten 4-Hydroxy-4-methyl-tetrahydropyrane (VI) siedende Verbindungen abzieht, als wenigstens ein Seitenabzug zumindest einen Teil der 2-substituierten 4-Hydroxy-4-methyl-tetrahydropyrane (VI) abzieht und im Sumpf oder im unteren Bereich der unteren Vereinigungssäule die 2-substituierten 4-Hydroxy-4-methyl-tetrahydropyrane (VI) abzieht, die nicht als Seitenabzug abgezogen werden und die höher als die 2-substituierten 4-Hydroxy-4-methyl-tetrahydropyrane (VI) siedenden Verbindungen abzieht.

In einer bevorzugten Ausführungsform enthält der aus der Abzugssäule am Kopf oder im oberen Bereich entnommene Abzug:
- zumindest einen Teil oder die Gesamtmenge der in dem Reaktionsprodukt aus Schritt a1) enthaltenen Dioxanverbindung (II),
- zumindest einen Teil oder die Gesamtmenge der in dem Reaktionsprodukt aus Schritt a1) enthaltenen Verbindungen (III.1), (III.2) und (III.3),
- falls vorhanden, nicht umgesetztes 3-Methylbut-3-en-1-ol der Formel (IV),
- falls vorhanden, nicht umgesetzten Aldehyd (V),
- geringe Mengen oder keine 4-Hydroxy-4-methyl-tetrahydropyrane (VI),
- Wasser.

In einer besonders bevorzugten Ausführungsform werden zur Umsetzung in Schritt a1) 3-Methylbut-3-en-1-ol der Formel (IV) und Isovaleraldehyd (V) eingesetzt. Dann enthält der aus der Abzugssäule am Kopf oder im oberen Bereich entnommene Abzug:
- zumindest einen Teil oder die Gesamtmenge der in dem Reaktionsprodukt aus Schritt a) enthaltenen Dioxanverbindung (II), worin R¹ für Isobutyl steht,
- zumindest einen Teil oder die Gesamtmenge der in dem Reaktionsprodukt aus Schritt a) enthaltenen Verbindungen (III.1), (III.2) und (III.3), worin R¹ für Isobutyl steht,
- falls vorhanden, nicht umgesetztes 3-Methylbut-3-en-1-ol der Formel (IV),
- falls vorhanden, nicht umgesetzten Isovaleraldehyd (V.a),
- geringe Mengen oder kein 2-(2-Methylpropyl)-4-hydroxy-4-methyl-tetrahydropyran der Formel (VI.a),
- Wasser.

Das so erhaltene Kopfprodukt kann einer Phasentrennung zur Abtrennung der Hauptmenge des Wassers unterzogen werden. Abgesehen von einer solchen Phasentrennung kann das so erhaltene Kopfprodukt in der Regel ohne weitere Aufarbeitung zur Umsetzung in Schritt b) eingesetzt werden. Gewünschtenfalls kann das Kopfprodukt einer weiteren Aufarbeitung zur Abtrennung wenigstens eines Teils der von den Verbindungen (III.1), (III.2), (III.3) verschiedenen Komponenten unterzogen werden. Dazu kann das Kopfprodukt z. B. einer weiteren destillativen Auftrennung unterzogen werden.

In einer bevorzugten Ausführungsform wird aus der Abzugssäule ein Seitenstrom oder werden aus der Abzugssäule zwei Seitenströme abgezogen. In einer speziellen Ausführung wird aus der Abzugssäule nur ein Seitenstrom abgezogen.

Werden in Schritt a2) mehrere Abzüge entnommen, die 2-substituierte 4-Hydroxy-4-methyl-tetrahydropyrane (I) enthalten, z. B. zwei verschiedene Seitenabzüge oder ein Seitenabzug und ein Sumpfabzug, so unterscheiden sich diese in der Regel hinsichtlich der Zusammensetzung der Stereoisomeren. Somit gelingt die Isolierung einer gegenüber dem Reaktionsprodukt aus Schritt a1) an cis-Diastereomeren angereicherten Fraktion und einer an trans-Diastereomeren angereicherten Fraktion. Bei ausreichender Trennleistung der eingesetzten Destillationsvorrichtung kann gewünschtenfalls zumindest eines der Diastereomeren in reiner Form erhalten werden.

Die Zulaufsäule, Abzugssäule, obere Vereinigungssäule und untere Vereinigungssäule können diskrete Bauelemente sein oder als Abschnitt oder Kammer einer Destillationskolonne ausgebildet sein, die mehrere Funktionen vereint. Der Ausdruck "kommunizierende Kolonnen" bedeutet, dass zwischen ihnen ein Austausch sowohl von aufsteigenden Brüden als auch von ablaufendem Kondensat erfolgt.

In einer bevorzugten Ausführungsform des erfindungsgemäßen Verfahrens erfolgt die destillative Auftrennung in Schritt a2) in einer Anordnung von Destillationskolonnen, die eine Trennwandkolonne oder eine Zusammenschaltung von mindestens zwei thermisch gekoppelten konventionellen Destillationskolonnen umfasst.

Trennwandkolonnen sind spezielle Destillationskolonnen mit mindestens einer Zulaufstelle und mindestens drei Entnahmestellen, bei denen sich zwischen Verdampfer und Kondensator der sogenannte Rektifizierbereich befindet, in dem ein Teil des im Kondensator gebildeten Kondensats sich flüssig als Rücklauf im Gegenstrom zu den aus der Verdampfungseinrichtung aufsteigenden Dämpfen abwärts bewegt und welche in einem Teilbereich der Kolonne unterhalb und/oder oberhalb der Zulaufstelle mindestens eine in Längsrichtung wirkende Trenneinrichtung (Trennwand) zur Verhinderung einer Quervermischung von Flüssigkeits- und/oder Brüdenstrom (Dampfstrom) enthält und welche somit eine destillative Trennung von Stoffgemischen ermöglichen. Das Grundprinzip der Trennwandkolonnen ist seit langem bekannt und beispielsweise in der US 2,471,134, in der EP-A-0 122 367 oder in G. Kaibel, Chem. Eng. Technol. Vol. 10, 1987, S. 92 bis 98 beschrieben.

Der allgemeine Grundaufbau einer Trennwandkolonne umfasst mindestens eine seitliche Zulaufstelle auf einer Seite der Trennwand und mindestens drei Entnahmestellen, von denen sich mindestens eine jenseits der Trennwand befindet. Da bei dieser Bauart im Bereich der Trennwand eine Quervermischung von Flüssigkeits- und/oder Brüdenstrom verhindert wird, ist es möglich, die Seitenprodukte in reiner Form zu erhalten. Hierdurch verringert sich bei der Auftrennung von Vielstoffgemischen im Allgemeinen die Zahl der insgesamt benötigten Destillationskolonnen. Zudem können beim Einsatz von Trennwandkolonnen gegenüber einer einfachen Hintereinanderschaltung zweier konventioneller Destillationskolonnen Investitionskosten sowie Energie eingespart werden (siehe M. Knott, Process Engineering, Vol. 2, 1993, February, Seite 33 bis 34).

Als konventionelle Destillationskolonnen werden im Sinne der Erfindung alle Destillationskolonnen bezeichnet, welche keine Trennwand enthalten. Bei thermisch gekoppelten konventionellen Destillationskolonnen werden Massen- und Energieströme wechselseitig ausgetauscht. Somit ist gegenüber einer einfachen Hintereinanderschaltung konventioneller Destillationskolonnen eine deutliche Einsparung von Energie möglich. Bevorzugt als Alternative zur Trennwandkolonne ist eine Verschaltung zweier thermisch gekoppelter Destillationskolonnen. Eine Übersicht verschiedener Anordnungen ist beispielsweise in G. Kaibel et al., Chem.-Ing.-Tech., Vol. 61, 1989, S. 16 bis 25 und G. Kaibel et al., Gas Separation & Purification, Vol. 4, 1990, June, S. 109 bis 114, gegeben.

In einer ersten bevorzugten Ausführungsform verwendet man zur Destillation eine Destillationskolonne mit einer thermisch gekoppelten Vorkolonne, d. h. die Abzugssäule, die obere Vereinigungssäule und die untere Vereinigungssäule sind als einstückige Destillationskolonne ausgebildet, und die Zulaufsäule ist als Vorkolonne zur Destillationskolonne ausgebildet. In einer zweiten bevorzugten Ausführungsform verwendet man eine Destillationskolonne mit einer thermisch gekoppelten Nachkolonne, d. h. die Zulaufsäule, die obere Vereinigungssäule und die untere Vereinigungssäule sind als einstückige Destillationskolonne ausgebildet und die Abzugssäule ist als Nachkolonne zu der Destillationskolonne ausgebildet. Destillationskolonnen mit beigeschalteten Hilfskolonnen sind bekannt und z. B. in Chem. Eng. Res. Des., Part A: Trans IChemE, März 1992, S. 118-132, "The design and optimisation of fully thermally coupled distillation columns", beschrieben.

Es hat sich als günstig erwiesen, aus dem Reaktionsprodukt aus Schritt a1) vor dem Einführen in die Zulaufsäule zumindest einen Teil der leichter als die 2-substituierten 4-Hydroxy-4-methyl-tetrahydropyrane (VI) siedenden Verbindungen zu entfernen. In einer speziellen Ausführungsform wird daher zur destillativen Auftrennung des Reaktionsprodukts aus Schritt a) eine Anordnung von Destillationskolonnen eingesetzt, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

Bevorzugt wird zur destillativen Auftrennung in Schritt a2)
a21) das Reaktionsgemisch aus Schritt a1) zunächst einer Auftrennung in der konventionellen Destillationskolonne unterzogen, wobei ein erstes Kopfprodukt erhalten wird, das an der Dioxanverbindung (II) und an den Verbindungen (III.1), (III.2) und (III.3) angereichert ist und im Wesentlichen keine Verbindungen der allgemeinen Formel (VI) enthält, und ein erstes Sumpfprodukt erhalten wird, das an den Verbindungen (III.1), (III.2) und (III.3) und der Dioxanverbindung (II) abgereichert ist und die Hauptmenge der Verbindungen der allgemeinen Formel (VI) enthält,
a22) das erste Sumpfprodukt aus Schritt a21) einer Auftrennung in der Trennwandkolonne oder in der Zusammenschaltung von zwei thermisch gekoppelten konventionellen Destillationskolonnen unterzogen, wobei ein zweites Kopfprodukt erhalten wird, das die nicht im ersten Kopfprodukt enthaltenen Verbindungen (III.1), (III.2), (III.3) und (II) sowie gegebenenfalls geringe Mengen der Verbindungen der allgemeinen Formel (VI) enthält, ein Seitenstrom erhalten wird, der im Wesentlichen aus Verbindung der allgemeinen Formel (VI) besteht und ein zweites Sumpfprodukt erhalten wird, das die Verbindungen der allgemeinen Formel (VI) enthält, die nicht im Kopfprodukt und nicht im Seitenstrom enthalten sind,
wobei das erste Kopfprodukt und/oder das zweite Kopfprodukt als Ausgangsmaterial in Schritt b) eingesetzt werden.

Bevorzugt steht auch in der zuvor genannten Ausführungsform in den Verbindungen der Formeln (II), (III.1), (III.2), (III.3) und (VI) R¹ für Isobutyl.

Der Ausdruck, wonach das erste Kopfprodukt im Wesentlichen keine Verbindungen der allgemeinen Formel (VI) enthält, bedeutet, dass der Anteil Verbindungen der allgemeinen Formel (VI) am ersten Kopfprodukt höchstens 5 Gew.-%, besonders bevorzugt höchstens 2 Gew.-%, insbesondere höchstens 1 Gew.-%, speziell höchstens 0,1 Gew.-%, bezogen auf das Gesamtgewicht des ersten Kopfprodukts, beträgt. In einer speziellen Ausführung enthält das erste Kopfprodukt keine Verbindungen der allgemeinen Formel (VI).

Das zweite Kopfprodukt kann beispielsweise 1 bis40 Gew.-%, besonders bevorzugt 2 bis 30 Gew.-%, insbesondere 5 bis 25 Gew.-%, speziell 10 bis 20 Gew.-%, Verbindungen der allgemeinen Formel (VI), bezogen auf das Gesamtgewicht des zweiten Kopfprodukts, enthalten.

In einer speziellen Ausführung besteht der Seitenstrom nur aus Verbindungen der allgemeinen Formel (VI).

Alternativ kann das zweite Sumpfprodukt Verbindungen enthalten, die höher sieden als die Verbindungen der allgemeinen Formel (VI).

Vorzugsweise wird nach dieser Ausführungsform das erste Kopfprodukt (insbesondere die an Wasser abgereicherte organische Phase des ersten Kopfprodukts) und/oder das zweite Kopfprodukt zur Umsetzung in Gegenwart einer starken säure und/oder eines sauren Ionenaustauschers Schritt b) eingesetzt. Dabei ist es unkritisch, wenn das zweite Kopfprodukt noch geringe Mengen der Verbindungen der allgemeinen Formel (VI) enthält, da diese die Umsetzung in Schritt b) nahezu unverändert durchlaufen und anschließend gewünschtenfalls abgetrennt und verwertet werden können.

In der Regel werden sich bei dieser Ausführungsform das Seitenprodukt und das zweite Sumpfprodukt bezüglich des Anteils der Stereoisomeren der Verbindungen der Formel (VI) unterscheiden.

### Schritt b)

In Schritt b) des erfindungsgemäßen Verfahrens wird das in Schritt a) bereitgestellte Ausgangsmaterial einer Umsetzung in Gegenwart einer starken Säure und/oder eines sauren Ionenaustauschers unterzogen, wobei ein gegenüber dem Ausgangsmaterial an der Dioxanverbindung der Formel (II) abgereichertes und an wenigstens einer der Verbindungen der Formeln (III.1), (III.2) oder (III.3) sowie an der Verbindung VI) 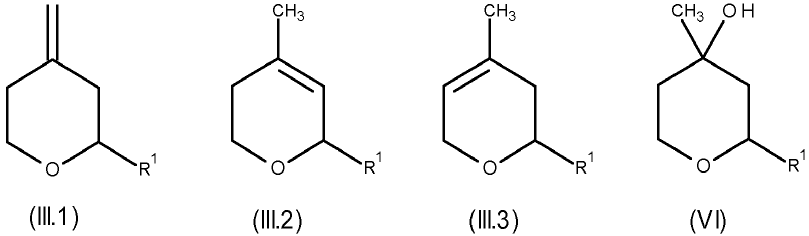 angereichertes Produktgemisch erhalten wird, wobei R¹ die zuvor angegebene Bedeutung hat.

Geeignete Säuren für die Umsetzung in Schritt b) sind prinzipiell die in Schritt a1) genannten sauren Katalysatoren. Auf die geeigneten und bevorzugten Ausführungsformen dieser sauren Katalysatoren wird hier in vollem Umfang Bezug genommen.

Prinzipiell kann für die Umsetzung in Schritt b) jede Substanz verwendet werden, die Brönstedt- oder Lewis-Acidität aufweist. Beispiele für geeignete Katalysatoren sind Protonensäuren, saure molekulare Elementverbindungen, saure Ionentauscher und Mischungen davon.

Bevorzugt erfolgt die Umsetzung in Schritt b) in Gegenwart einer Säure, die ausgewählt ist unter Salzsäure, Schwefelsäure, Methansulfonsäure, p-Toluolsulfonsäure und stark sauren Kationenaustauschern.

In einer speziellen Ausführungsform wird die Umsetzung in Schritt b) in Gegenwart eines stark sauren Kationenaustauschers oder Methansulfonsäure durchgeführt.

In einer ersten Variante erfolgt die Umsetzung in Schritt b) in Gegenwart einer Brönstedt-Säure, die vorzugsweise ausgewählt ist unter Salzsäure, Schwefelsäure, Methansulfonsäure, p-Toluolsulfonsäure.

In dieser ersten Variante beträgt der Wassergehalt des Reaktionsgemisches 0 bis 10 Gew.-%, besonders bevorzugt 1 bis 5 Gew.-%, bezogen auf das Gesamtgewicht des Reaktionsgemisches.

Bevorzugt in dieser ersten Variante wird die Brönstedt-Säure in einer Menge von 1 bis 5 Mol-%, besonders bevorzugt von 1 bis 3 Mol-% Säuregruppen der Brönstedt-Säure, bezogen auf die Dioxanverbindung der allgemeinen Formel (II) eingesetzt. Gegebenenfalls wird in dieser ersten Variante in Schritt b) ein Lösungsmittel eingesetzt, das vorzugsweise ausgewählt ist unter Kohlenwasserstoffen und Kohlenwasserstoffgemischen. Geeignete Lösungsmittel sind beispielsweise Hexan, Heptan, Ligroin, Petrolether, Cyclohexan, Dekalin, Toluol, Xylol und Mischungen davon. Bevorzugt wird kein Lösungsmittel eingesetzt.

Bevorzugt erfolgt die Umsetzung in Schritt b) nach dieser ersten Variante bei einer Temperatur im Bereich von 20 bis 180 °C, besonders bevorzugt 50 bis 140 °C.

In einer zweiten Variante erfolgt die Umsetzung in Schritt b) in Gegenwart eines stark sauren Kationenaustauschers. Geeignete stark saure Kationenaustauscher sind die zuvor in Schritt a1) beschriebenen, worauf hier in vollem Umfang Bezug genommen wird.

Die stark sauren lonentauscherharze werden in der Regel mit Salzsäure und/oder Schwefelsäure regeneriert.

Bevorzugt erfolgt die Umsetzung in Schritt b) in Gegenwart eines stark sauren Kationenaustauschers und in Gegenwart von Wasser. Üblicherweise führt man die Umsetzung in Schritt b) dann in Gegenwart von 1 bis 10 Gew.-% Wasser, besonders bevorzugt von 2 bis 5 Gew.-% Wasser, bezogen auf das Gesamtgewicht des Reaktionsgemischs durch.

Die Menge an stark saurem Kationenaustauscher in Schritt b) ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Üblicherweise setzt man den stark sauren Kationentauscher in einer Menge von etwa 0,1 bis etwa 5 Gew.-%, bevorzugt in einer Menge von etwa 0,5 bis etwa 3 Gew.-% und besonders bevorzugt in einer Menge von etwa 1 bis etwa 3 Gew.-%, jeweils bezogen auf das Gesamtgewicht des Reaktionsgemischs ein. Dabei beziehen sich die Angaben auf den gebrauchsfertigen Kationenaustauscher, der in der Regel mit Wasser vorbehandelt wird und dementsprechend Mengen von bis zu etwa 70 Gew.-%, bevorzugt von etwa 30 bis etwa 65 Gew.-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen. Die genannten stark sauren Kationenaustauscher können in Schritt b) sowohl einzeln oder auch in Form von Gemischen eingesetzt werden.

Die Umsetzung in Schritt b) in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a) in Gegenwart eines stark sauren Kationenaustauschers ohne Zusatz eines organischen Lösungsmittels durch.

Bevorzugt wird die Umsetzung des in Schritt a) bereitgestellten Ausgangsmaterials in Schritt b) in Gegenwart von Wasser und in Gegenwart eines stark sauren Kationentauschers bei einer Temperatur im Bereich von 10 bis 200 °C, besonders bevorzugt bei einer Temperatur im Bereich von 50 bis 180 °C und insbesondere bei einer Temperatur im Bereich von 80 bis 150 °C durchgeführt.

Die Umsetzung in Schritt b) kann diskontinuierlich oder kontinuierlich durchgeführt werden. Dabei kann man beispielsweise im diskontinuierlichen Fall die Umsetzung so vornehmen, dass man das in Schritt a) bereitgestellte Ausgangsmaterial, gegebenenfalls Wasser und gegebenenfalls ein organisches Lösungsmittel in einem geeigneten Reaktionsgefäß vorlegt und den sauren Katalysator zugibt. Nach Abschluss der Reaktion kann dann die starke Säure durch geeignete Trennverfahren vom erhaltenen Reaktionsgemisch abgetrennt werden.

Wird in Schritt b) eine Brönstedt-Säure, die vorzugsweise ausgewählt ist unter Salzsäure, Schwefelsäure, Methansulfonsäure, p-Toluolsulfonsäure, eingesetzt, so kann die Abtrennung der Säure z. B. direkt oder nach wässriger Aufarbeitung destillativ erfolgen.

Wird in Schritt b) ein stark saurer Kationenaustauscher eingesetzt, so kann die Abtrennung z. B. durch Filtration oder durch Zentrifugation erfolgen.

In einer geeigneten Ausführungsform führt man die Umsetzung in Schritt b) kontinuierlich durch. Dazu kann beispielsweise eine Mischung des in Schritt a) bereitgestellten Ausgangsmaterials mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und das Ausgangsmaterial und das Wasser in diesen kontinuierlich eingetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden.

Die Umsetzung in Schritt b) wird in der Regel fortgeführt, bis der Gehalt des Reaktionsgemischs an der Dioxanverbindung der allgemeinen Formel (II) unter dem angestrebten Höchstwert liegt.

Vorzugsweise enthält das in Schritt b) erhaltene Reaktionsgemisch die Dioxan-Verbindung der Formel (II) in einer Gesamtmenge von höchstens 5 Gew.-%, besonders bevorzugt von höchstens 2 Gew.-%, insbesondere von höchstens 1 Gew.-%, bezogen auf das Gesamtgewicht des Reaktionsgemischs.

Vorzugsweise werden durch die Umsetzung in Schritt b) 5 bis 50 Gew.-%, besonders bevorzugt 10 bis 40 Gew.-%, insbesondere 15 bis 30 Gew.-% der in dem Ausgangsmaterial enthaltenen Dioxan-Verbindung der Formel (II) in Verbindungen der Formeln (III.1), (III.2) und (III.3) umgewandelt.

Vorzugsweise enthält das in Schritt b) erhaltene Reaktionsgemisch die Verbindungen der Formeln (III.1), (III.2) und (III.3) in einer Gesamtmenge von 20 bis 80 Gew.-%, besonders bevorzugt 35 bis 65 Gew.-%, bezogen auf das Gesamtgewicht des Reaktionsgemischs.

Vorzugsweise werden durch die Umsetzung in Schritt b) 10 bis 70 Gew.-%, besonders bevorzugt 20 bis 60 Gew.-%, insbesondere 30 bis 50 Gew.-% der in dem Ausgangsmaterial enthaltenen Dioxan-Verbindung der Formel (II) in das 2-substituierte 4-Hydroxy-4-methyl-tetrahydropyran der Formel (VI) umgewandelt.

Vorzugsweise enthält das in Schritt b) erhaltene Reaktionsgemisch das 2-substituierte 4-Hydroxy-4-methyl-tetrahydropyran der Formel (VI) in einer Gesamtmenge von 10 bis 40 Gew.-%, besonders bevorzugt 15 bis 30 Gew.-%, bezogen auf das Gesamtgewicht des Reaktionsgemischs.

In einer typischen Zusammensetzung enthält das in Schritt b) erhaltene Reaktionsgemisch die folgenden Verbindungen, jeweils bezogen auf das Gesamtgewicht des Reaktionsgemischs:
Isovaleraldehyd: 0 - 15 Gew.-%,
Isoprenol: 0 - 5 Gew.-%,
Dioxanverbindung (II): 0 - 2 Gew.-%,
Verbindungen der Formeln (III.1), (III.2) und/oder (III.3): insgesamt 30 - 75 Gew.-%, von (II) verschiedene Acetale: 0 - 2 Gew.-%,
Verbindungen der Formel (VI): 10 - 35 Gew.-%.

### Schritt c)

In Schritt c) des erfindungsgemäßen Verfahrens wird das in Schritt b) erhaltene Produktgemisch einer Hydrierung unterzogen. Durch die Hydrierung in Schritt c) werden die Verbindungen (III.1), (III.2) und (III.3) zu den entsprechenden 2-substituierten 4-Methyl-tetrahydropyranen der allgemeinen Formel (I) umgewandelt.

Die Hydrierung in Schritt c) kann auf an sich herkömmliche Weise mit Hydrierkatalysatoren des Standes der Technik ausgeführt werden. Die Hydrierung kann katalytisch entweder in der Gas- oder Flüssigphase erfolgen. Vorzugsweise wird die Hydrierung in Schritt c) des erfindungsgemäßen Verfahrens in flüssiger Phase in Gegenwart eines heterogenen Hydrierkatalysators und eines wasserstoffhaltigen Gases durchgeführt.

Als Hydrierkatalysatoren kommen prinzipiell alle zur Hydrierung von ungesättigten organischen Verbindungen geeigneten homogenen und heterogenen Katalysatoren in Betracht. Dazu zählen z. B. Metalle, Metalloxide, Metallverbindungen oder Gemische davon. Geeignete Hydrierkatalysatoren enthalten vorzugsweise wenigstens ein Übergangsmetall, bevorzugt aus den Nebengruppen I. und VI. bis VIII. des Periodensystems der Elemente. Dazu zählen vorzugsweise Cu, Cr, Mo, Mn, Re, W, Fe, Rh, Co, Ni, Pd, Pt, Ru, Zn oder deren Mischungen.

Die Katalysatoren können allein aus den Aktivkomponenten bestehen, oder die Aktivkomponenten können auf Trägern aufgebracht sein. Als Trägermaterialien eignen sich z. B. Al₂O₃, SiO₂, ZrO₂, TiO₂, Aktivkohle, ZnO, BaO und MgO oder Mischungen daraus.

Zur Erhöhung der katalytischen Aktivität können Fe, Co und bevorzugt Ni auch in Form der Raney-Katalysatoren oder als Metallschwamm mit einer sehr großen Oberfläche verwendet werden.

Bevorzugt wird für die Hydrierung im Schritt c) des erfindungsgemäßen Verfahrens Raney-Nickel oder Raney-Cobalt eingesetzt. Des Weiteren kann vorteilhaft Palladium auf Kohlenstoff oder Platin auf Kohlenstoff eingesetzt werden.

Andere geeignete Katalysatoren enthalten z. B. 80 bis 100 Gew.-% Nickel und/oder Cobalt und bis zu 20 Gew.-% aktivierende Metalle wie Kupfer und/oder Chrom. Besonders vorteilhaft werden solche Katalysatoren als Trägerkatalysatoren verwendet. Der Gehalt an katalytisch aktiven Metallen solcher Trägerkatalysatoren beträgt in der Regel 5 bis 80 Gew.-%, bezogen auf die Summe von katalytisch aktiven Metallen und Träger.

Die Katalysatoren können zur Hydrierung in Schritt c) als Formkörper eingesetzt werden. Beispiele umfassen Katalysatorextrudate, wie Stränge Rippstränge und andere Extrudatformen, Schalenkatalysatoren, Tabletten, Ringe, Kugeln, Splitt, etc.

Bevorzugt wird die Hydrierung in Schritt c) bei einer Temperatur von 20 bis 220 °C, vorzugsweise 40 bis 200 °C, insbesondere 50 bis 180 °C, durchgeführt.

Sofern man die Umsetzung in der Gasphase durchführt, liegt der Druck vorzugsweise in einem Bereich von 1 bis 200 bar, besonders bevorzugt 10 bis 150 bar.

Sofern man die Umsetzung in der Flüssigphase durchführt, liegt der Druck vorzugsweise in einem Bereich von 2 bis 500 bar, besonders bevorzugt von 3 bis 300 bar, insbesondere von 4 bis 250 bar, speziell von 5 bis 200 bar.

Die Hydrierung in Schritt c) kann in einem Reaktor oder mehreren hintereinander geschalteten Reaktoren durchgeführt werden. Die Hydrierung kann diskontinuierlich oder kontinuierlich erfolgen. Zur diskontinuierlichen Hydrierung kann z. B. ein Druckgefäß eingesetzt werden. Geeignete Druckgefäße sind z. B. Autoklaven, die mit einer Vorrichtung zum Beheizen und zum Rühren des Reaktorinhalts ausgestattet sind. Bevorzugt erfolgt die Hydrierung in der Flüssigphase über einem Festbett, vorzugsweise in Sumpf- oder Rieselfahrweise oder in Form einer Suspensionskatalyse.

Die Hydrierung kann ohne oder mit Zusatz eines Lösungsmittels erfolgen. Als Lösungsmittel kommen Alkohole, Ether, Kohlenwasserstoffe, wie beispielsweise Methanol, Ethanol, Isopropanol, Dioxan, Tetrahydrofuran, n-Pentan, Hexan, Cyclohexan, Toluol, etc., in Frage. Bevorzugt erfolgt die Hydrierung in Schritt c) ohne Zusatz eines Lösungsmittels.

Zur Hydrierung in Schritt c) kann man das in Schritt b) erhaltene Produktgemisch mit einem wasserstoffhaltigen Gas und einem Hydrierkatalysator in Kontakt bringen. Geeignete wasserstoffhaltige Gase sind ausgewählt unter Wasserstoff und Gemischen von Wasserstoff mit wenigstens einem inerten Gas. Geeignete inerte Gase sind z. B. Stickstoff oder Argon. Bevorzugt wird zur Hydrierung in Schritt c) Wasserstoff in unverdünnter Form, üblicherweise in einer Reinheit von etwa 99,9 Vol.-%, eingesetzt.

Durch die Hydrierung in Schritt c) werden die im Ausgangsgemisch enthaltenen Verbindungen (III.1), (III.2) und (III.3) in 2-substituierte 4-Methyl-tetrahydropyrane (I) überführt. Bevorzugt enthält das zur Hydrierung eingesetzte Ausgangsgemisch Verbindungen der Formel (III.1), (III.2) und (III.3), wobei der Rest R¹ für Isobutyl steht. Dann werden durch die Hydrierung in Schritt c) die im Ausgangsgemisch enthaltenen Verbindungen (III.1), (III.2) und (III.3) in 2-Isobutyl-4-methyl-tetrahydropyran (I) (Dihydrorosenoxid) überführt.

Derartige Gemische eignen sich aufgrund ihrer besonderen Geruchseigenschaften in besonderem Maße zur Verwendung als Aromachemikalien, beispielsweise als Komponente mit rosenduftartigem Charakter zur Herstellung von Riechstoffkompositionen.

Nach einer speziellen Ausführungsform des erfindungsgemäßen Verfahrens werden die Schritte b) und c) zumindest teilweise zeitgleich durchgeführt. Dazu kann beispielsweise so vorgegangen werden, dass man die Umsetzung unter den Bedingungen von Schritt b) durchführt, bis der Gehalt des Reaktionsgemischs an der Dioxanverbindung der allgemeinen Formel (II) unter dem angestrebten Höchstwert liegt, anschließend das Reaktionsgemisch mit Wasserstoff beaufschlagt, gegebenenfalls einen Hydrierkatalysator zugibt und die Reaktion unter Hydrierbedingungen fortsetzt.

Nach einer weiteren speziellen Ausführungsform des erfindungsgemäßen Verfahrens werden die Schritte b) und c) zeitgleich durchgeführt, wobei das Reaktionsgemisch von Beginn an mit Wasserstoff beaufschlagt wird.

### Schritt d)

Das in Schritt c) erhaltene Hydrierprodukt zeichnet sich vorteilhafterweise durch einen gegenüber dem Ausgangsmaterial deutlich verringerten Gehalt an der Dioxanverbindung der allgemeinen Formel (II) aus. Es lässt sich durch einfache Reinigungsschritte in eine zur kommerziellen Verwendung geeignete Form überführen. Gewünschtenfalls kann das in Schritt c) erhaltene Hydrierprodukt einer weiteren Aufarbeitung unterzogen werden. Dazu kann das in Schritt c) erhaltene Hydrierprodukt prinzipiell üblichen, dem Fachmann bekannten Reinigungsverfahren unterzogen werden. Dazu zählt beispielsweise eine Destillation, eine Extraktion oder eine Kombination davon.

Bevorzugt wird aus dem in Schritt c) erhaltenen Hydrierprodukt eine an 2-substituierten 4-Methyl-tetrahydropyranen (I) angereicherte Fraktion und eine an 2-substituierten 4-Methyl-tetrahydropyranen (I) abgereicherte Fraktion isoliert. Bevorzugt ist die an 2-substituierten 4-Methyl-tetrahydropyranen (I) abgereicherte Fraktion an der Dioxanverbindung (II) und/oder dem 2-substituierten 4-Hydroxy-4-methyl-tetrahydropyran der allgemeinen Formel (VI) angereichert.

Bevorzugt wird das in Schritt c) erhaltene Hydrierprodukt einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc., und Kombinationen davon.

Bevorzugt wird das in Schritt c) erhaltene Hydrierprodukt in Schritt d) einer destillativen Auftrennung in wenigstens einer Destillationskolonne unterzogen, die mit trennwirksamen Einbauten versehen ist.

Bevorzugt wird in Schritt d) aus dem in Schritt c) erhaltenen Hydrierprodukt eine an 2-substituierten 4-Methyl-tetrahydropyranen (I) angereicherte Fraktion isoliert, wobei das Diastereomerenverhältnis des cis-Diastereomeren zum trans-Diastereomeren in einem Bereich von 60 zu 40 bis 100 zu 0, bevorzugt von 65 zu 35 bis 90 zu 10, liegt.

Besonders bevorzugt wird in Schritt d) aus dem in Schritt c) erhaltenen Hydrierprodukt eine an 2-Isobutyl-4-methyl-tetrahydropyran (I) angereicherte Fraktion isoliert, wobei das Diastereomerenverhältnis des cis-Diastereomeren zum trans-Diastereomeren in einem Bereich von 60 zu 40 bis 100 zu 0, bevorzugt von 65 zu 35 bis 90 zu 10, liegt.

Bevorzugt wird in Schritt d) aus dem in Schritt c) erhaltenen Hydrierprodukt eine an 2-substituierten 4-Methyl-tetrahydropyranen (I) angereicherte Fraktion isoliert, die einen Gehalt an Dioxanverbindungen der allgemeinen Formel (II), worin R¹ die zuvor angegebenen Bedeutungen hat und insbesondere für Isobutyl steht, von höchstens 2 Gew.-%, besonders bevorzugt von höchstens 1 Gew.-%, ganz besonders bevorzugt von höchstens 0,1 Gew%, aufweist.

Zur Entfernung von weiteren wasserlöslichen Verunreinigungen kann die in Schritt d) erhaltene an 2-substituierten 4-Methyl-tetrahydropyranen (I) angereicherte Fraktion wenigstens einem Waschschritt mit Wasser unterzogen werden. Alternativ oder zusätzlich kann die in Schritt d) erhaltene an 2-substituierten 4-Methyl-tetrahydropyranen (I) angereicherte Fraktion einer weiteren destillativen Aufreinigung unterzogen werden.

Die erfindungsgemäßen Zusammensetzungen und die nach dem erfindungsgemäßen Verfahren erhältlichen Zusammensetzungen eignen sich besonders vorteilhaft als Riechstoff oder zur Bereitstellung eines Riechstoffs.

Die erfindungsgemäßen Zusammensetzungen können für den Einsatz als Riechstoff mit wenigstens einem auf diesem Anwendungsgebiet üblichen Lösemittel beliebig verdünnt werden. Beispielhaft seien als geeignete Lösemittel genannt: Ethanol, Dipropylenglycol oder dessen Ether, Phthalate, Propylenglykole, oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die nach dem erfindungsgemäßen Verfahren erhaltenen Riechstoffe weisen auf Grund der strukturellen und chemischen Ähnlichkeit der Komponenten eine hohe Stabilität und Haltbarkeit auf. Die nach dem erfindungsgemäßen Verfahren erhältlichen Isomerengemische von 2-(2-Methylpropyl)-4-methyl-tetrahydropyran der Formel (la) (Dihydrorosenoxid) zeichnen sich durch einen angenehmen rosenartigem Charakter aus. Die nach dem erfindungsgemäßen Verfahren gegebenenfalls zusätzlich erhältlichen Isomerengemische von 2-(2-Methylpropyl)-4-hydroxy-4-methyl-tetrahydropyran der Formel (VIa) zeichnen sich durch einen angenehmen Maiglöckchengeruch aus.

Die nach dem erfindungsgemäßen Verfahren erhaltenen Riechstoffe eigenen sich zur Einarbeitung in kosmetische Zusammensetzungen sowie Gebrauchs- und Verbrauchsgüter bzw. Mittel wie sie im Folgenden näher beschrieben sind, wobei die Riechstoffe in die genannten Güter eingearbeitet oder auch auf solche aufgebracht sein können. Unter einer organoleptisch wirksamen Menge ist dabei wie im Rahmen der gesamten vorliegenden Erfindung insbesondere eine solche Menge zu verstehen, die bei sachgemäßer Anwendung ausreicht, beim Anwender bzw. Verbraucher einen Dufteindruck hervorzurufen.

Als kosmetische Zusammensetzungen sind alle üblichen kosmetischen Zusammensetzungen geeignet. Dabei handelt es sich bevorzugt um Parfum, Eau de Toilette, Deodorants, Seife, Duschgel, Badegel, Cremes, Lotions, Sonnenschutzmittel, Zusammensetzungen zur Reinigung und Pflege der Haare wie Haarshampoo, Spülung, Haargel, Haarfestiger in Form von Flüssigkeiten oder Schäumen und weitere Reinigungs- oder Pflegemittel für die Haare, Zusammensetzungen zur dekorativen Anwendung am menschlichen Körper, wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Die nach dem erfindungsgemäßen Verfahren erhaltenen Riechstoffe eignen sich speziell für einen Einsatz in Parfums, z. B. als Eau de Toilette, Duschgele, Badegele und Körperdeodorants.

Sie eignen sich weiterhin zur Aromatisierung von Verbrauchs- oder Gebrauchsgütern, in die sie eingearbeitet bzw. auf die sie aufgebracht werden und ihnen dadurch einen angenehmen frischen grünen Akzent verleihen. Beispiele für Verbrauchs- oder Gebrauchsgüter sind: Raumluftdeodorants (Air Care), Reinigungsmittel oder Pflegemittel für Textilien (speziell Waschmittel, Weichspüler), Textilbehandlungsmittel wie beispielsweise Bügelhilfsmittel, Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Bleichen, Toilettensteine, Entkalkungsmittel, Düngemittel, Baustoffe, Schimmelentferner, Desinfektionsmittel, Produkte für die Auto- bzw. Fahrzeugpflege und dergleichen mehr.

Die folgenden Beispiele dienen der Verdeutlichung der Erfindung, ohne sie in irgendeiner Weise zu beschränken.

### BEISPIELE

Gaschromatographische Analysen wurden nach der folgenden Methode durchgeführt:
Säule: DB WAX 30 m x 0,32 mm;
FD 0,25 µm;

| | |
|---|---|
| Injektortemperatur: | 200 °C; Detektortemperatur 280 °C; |
| Temperaturprogramm: | Anfangstemp.: 50 °C, mit 3 °C/min auf 170 °C, mit 20 °C/min auf 230 °C, 7 Min isotherm; |
| Retentionszeiten: | Isovaleraldehyd t_{R} = 3,7 min |
| | cis-Dihydrorosenoxid t_{R} = 8,4 min |
| | trans-Dihydrorosenoxid t_{R} = 9,6 min |
| | 4,4-Dimethyl-2-isobutyl-1,3-dioxan t_{R} = 11,9 min |

Konzentrationen der erhaltenen Rohprodukte (Gew.-%) wurden GC-analytisch mit internem Standard ermittelt.

### Beispiel 1:

### (Umsetzung von 4,4-Dimethyl-2-isobutyl-1,3-dioxan mit einem sauren Ionenaustauscher)

4,4-Dimethyl-2-isobutyl-1,3-dioxan (30 g, Reinheit > 99 %) wurde vorgelegt, mit dem sauren Ionenaustauscher Amberlyst 131 H wet (6 g, 20 Gew.-%, bezogen auf 4,4-Dimethyl-2-isobutyl-1,3-dioxan) versetzt und unter Rühren auf Rückfluss (86 bis 92 °C) erhitzt. Nach einer Reaktionszeit von 20 h wurde ein Rohprodukt mit folgender Zusammensetzung erhalten: Isovaleraldehyd: 14,9 GC-Gew.-% (t_{R} = 3.6 min); Dihydropyrane (III.1 - III.3): 15,2 GC-Gew.-% (t_{R} = 9,4, 11,0, 11,5 min); Isoprenol: 0,7 GC-Gew.-% (t_{R} = 9,7 min); 4,4-Dimethyl-2-isobutyl-1,3-dioxan: 13,8 GC-Gew.-% (t_{R} = 11,3 min); 2-Isobutyl-4-hydroxy-4-methyl-tetrahydropyran: 35,4 GC-Gew.-% (t_{R} = 27,2, 28,6 min).

### Beispiel 2:

### (Umsetzung der bei der Auftrennung eines Reaktionsgemisches aus der Umsetzung von Isovaleraldehyd und Isoprenol in einer Trennwandkolonne isolierten Leichtsieder):

Eine Mischung (gesamt = 2000 g) aus Isovaleraldehyd (0,5 %), Isoprenol (7,7 %), den isomeren Dihydropyranen der Formeln (III.1), (III.2) und (III.3) (43,5 %), 4,4-Dimethyl-2-isobutyl-1,3-dioxan (29,7 %) und 2-Isobutyl-4-hydroxy-4-methyl-tetrahydropyran (17,1 %) wurde in einem Autoklaven (max. Befüllung 3500 ml) vorgelegt und mit Amberlyst 131 H wet (40 g, 2 Gew.-%, bezogen auf die eingesetzte Reaktionsmischung) versetzt. Nach dem Schließen des Autoklaven wurde dieser auf 120 °C aufgeheizt und bei dieser Temperatur für 10 h gerührt. Nach Abkühlen auf Raumtemperatur wurde der Austrag über einer Filternutsche (Nennweite der Poren 10 bis 16 µm) filtriert. Man erhielt das Rohprodukt mit folgender Zusammensetzung: Isovaleraldehyd: 7,0 GC-Gew.-% (t_{R} = 3,6 min); Dihydropyrane (III.1 - III.3): 51,2 GC-Gew.-% (t_{R} = 9,3, 11,1, 11,6 min); Isoprenol: 0,3 GC-Gew.-% (t_{R} = 9,9 min); 4,4-Dimethyl-2-isobutyl-1,3-dioxan: 0,7 GC-Gew.-% (t_{R} = 11,3 min); 2-Isobutyl-4-hydroxy-4-methyl-tetrahydropyran: 21,2 GC-Gew.-% (t_{R} = 27,2 min, 28,5 min).

### Beispiel 3:

### (Hydrierung des Austrags der sauren Umsetzung aus Beispiel 2)

Eine Mischung (gesamt = 1700 g) aus Isovaleraldehyd (7,0 %), den isomeren Dihydropyranen der Formeln (III.1), (III.2) und (III.3) (51,2 %), Isoprenol (0,3 %), 4,4-Dimethyl-2-isobutyl-1,3-dioxan (0,7 %) und Pyranol (21,2 %) wurde in einem Autoklaven (max. Befüllung 3500 ml) vorgelegt und mit Raney-Nickel-Katalysator (wasserfeucht; 18 g) versetzt. Nach dem Schließen des Autoklaven wurde dieser drei Mal mit Stickstoff (20 bar) gespült, der Rührer eingeschaltet (700 U/min), mit Wasserstoff ein Druck von 10 bar aufgepresst und der Autoklav auf 150 °C aufgeheizt. Bei 150 °C wurden 70 bar Wasserstoff aufgepresst und bei diesem Druck für weitere 6 h gerührt. Nach Abkühlen auf Raumtemperatur und Entspannen auf 0 bar wurde der Austrag über einer Filternutsche (Nennweite der Poren 10 bis 16 µm) filtriert. Man erhielt das Rohprodukt mit folgender Zusammensetzung: Isovaleraldehyd: 1,2 GC-Gew.-% (tR = 3,7 min); cis-Dihydrorosenoxid: 23,1 GC-Gew.-% (tR = 7,9 min); Isoamylalkohol: 6,7 GC-Gew.-% (tR = 8,6 min); trans-Dihydrorosenoxid: 25,3 GC-Gew.-% (tR = 9,1 min); 4,4-Dimethyl-2-isobutyl-1,3-dioxan: 1,1 GC-Gew.-% (tR = 11,2 min); 2-Isobutyl-4-hydroxy-4-methyl-tetrahydropyran: 21,0 GC-Gew.-% (tR = 27,2, 28,5 min).

### Beispiel 4:

### (Umsetzung der bei der Auftrennung eines Reaktionsgemisches aus der Umsetzung von Isovaleraldehyd und Isoprenol in einer Trennwandkolonne isolierten Leichtsieder)

Eine Mischung (gesamt = 100 g) aus Isovaleraldehyd (0,4 %), Isoprenol (0,3 %), den isomeren Dihydropyranen der Formeln (III.1), (III.2) und (III.3) (67,8 %), 4,4-Dimethyl-2-isobutyl-1,3-dioxan (18,2 %) und 2-Isobutyl-4-hydroxy-4-methyl-tetrahydropyran (13,0 %) wurde in einem Autoklaven (max. Befüllung 300 ml) vorgelegt und mit Amberlyst 131 H wet (1 g, 1 Gew.-%, bezogen auf die eingesetzte Reaktionsmischung) versetzt. Nach dem Schließen des Autoklaven wurde dieser auf 130 °C aufgeheizt und bei dieser Temperatur für 10 h gerührt. Nach Abkühlen auf Raumtemperatur wurde der Austrag über einer Filternutsche (Nennweite der Poren 10 bis 16 µm) filtriert. Man erhielt das Rohprodukt mit folgender Zusammensetzung: Isovaleraldehyd: 3,1 GC-Gew.-% (t_{R} = 3,6 min); Dihydropyrane (III.1 - III.3): 66,3 GC-Gew.-% (t_{R} = 9,4, 11,2, 11,7 min); Isoprenol: 0,4 GC-Gew.-% (t_{R} = 9,9 min); 4,4-Dimethyl-2-isobutyl-1,3-dioxan: 0,1 GC-Gew.-% (t_{R} = 11,4 min); 2-Isobutyl-4-hydroxy-4-methyl-tetrahydropyran: 11,6 GC-Gew.-% (t_{R} = 27,2 min, 28,6 min).

### Beispiel 5:

### (Hydrierung des Austrags der sauren Umsetzung aus Beispiel 4)

Eine Mischung (gesamt = 60 g) aus Isovaleraldehyd (3,1 %), den isomeren Dihydropyranen der Formeln (III.1), (III.2) und (III.3) (66,3 %), Isoprenol (0,4 %), 4,4-Dimethyl-2-isobutyl-1,3-dioxan (0,1 %) und 2-Isobutyl-4-hydroxy-4-methyl-tetrahydropyran (11,6 %) wurde in einem Autoklaven (max. Befüllung 300 ml) vorgelegt und mit Raney-Nickel-Katalysator (wasserfeucht; 0,6 g) versetzt. Nach dem Schließen des Autoklaven wurde dieser drei Mal mit Stickstoff (20 bar) gespült, der Rührer eingeschaltet (700 U/min), mit Wasserstoff ein Druck von 10 bar aufgepresst und der Autoklav auf 150 °C aufgeheizt. Bei 150 °C wurden 70 bar Wasserstoff aufgepresst und bei diesem Druck für weitere 10 h gerührt. Nach Abkühlen auf Raumtemperatur und Entspannen auf 0 bar wurde der Austrag über einer Filternutsche (Nennweite der Poren 10 bis 16 µm) filtriert. Man erhielt das Rohprodukt mit folgender Zusammensetzung: cis-Dihydrorosenoxid: 33,9 GC-Gew.-% (tR = 7,9 min); Isoamylalkohol: 2,6 GC-Gew.-% (tR = 8,6 min); trans-Dihydrorosenoxid: 32,6 GC-Gew.-% (tR = 9,2 min); 4,4-Dimethyl-2-isobutyl-1,3-dioxan: 0,5 GC-Gew.-% (tR = 11,2 min); 2-Isobutyl-4-hydroxy-4-methyl-tetrahydropyran: 11,7 GC-Gew.-% (tR = 27,2, 28,6 min).

### Beispiel 6:

### (saure Umsetzung und Hydrierung in einer Stufe)

Eine Mischung (gesamt = 100 g) aus Isovaleraldehyd (11,8 %), den isomeren Dihydropyranen der Formeln (III.1), (III.2) und (III.3) (48,7 %), Isoprenol (10,5 %), 4,4-Dimethyl-2-isobutyl-1,3-dioxan (20,4 %) und 2-Isobutyl-4-hydroxy-4-methyl-tetrahydropyran (6,0 %) wurde in einem Autoklaven (max. Befüllung 300 ml) vorgelegt und mit Amberlyst 131 H wet (10 g, 10 Gew.-%, bezogen auf die eingesetzte Reaktionsmischung) und mit Raney-Nickel-Katalysator (wasserfeucht; 1,0 g) versetzt. Nach dem Schließen des Autoklaven wurde dieser drei Mal mit Stickstoff (20 bar) gespült, der Rührer eingeschaltet (700 U/min), mit Wasserstoff ein Druck von 10 bar aufgepresst und der Autoklav auf 130 °C aufgeheizt. Bei 130 °C wurden 50 bar Wasserstoff aufgepresst und bei diesem Druck für weitere 20 h gerührt. Nach Abkühlen auf Raumtemperatur und Entspannen auf 0 bar wurde der Austrag über einer Filternutsche (Nennweite der Poren 10 bis 16 µm) filtriert. Man erhielt das Rohprodukt mit folgender Zusammensetzung: Isovaleraldehyd: 2,5 GC-Gew.-% (t_{R} = 3,7 min); cis-Dihydrorosenoxid: 18,8 GC-Gew.-% (tR = 7,9 min); Isoamylalkohol: 12,7 GC-Gew.-% (tR = 8,7 min); trans-Dihydrorosenoxid: 28,4 GC-Gew.-% (tR = 9,2 min); 4,4-Dimethyl-2-isobutyl-1,3-dioxan: 0,2 GC-Gew.-% (tR = 11,3 min); 2-Isobutyl-4-hydroxy-4-methyl-tetrahydropyran: 6,0 GC-Gew.-% (tR = 27,2, 28,6 min).

### Beispiel 7:

### (Umsetzung der bei der Auftrennung eines Reaktionsgemisches aus der Umsetzung von Isovaleraldehyd und Isoprenol in einer Trennwandkolonne isolierten Leichtsieder)

Eine Mischung (gesamt = 60 g) aus Isovaleraldehyd (0,3 %), Isoprenol (0,3 %), den isomeren Dihydropyranen der Formeln (III.1), (III.2) und (III.3) (66,1 %), 4,4-Dimethyl-2-isobutyl-1,3-dioxan (17,8 %) und 2-Isobutyl-4-hydroxy-4-methyl-tetrahydropyran (12,7 %) wurde mit Amberlyst 131 H wet (3 g, 5 Gew.-%, bezogen auf die eingesetzte Reaktionsmischung) versetzt. Anschließend wurde auf Rückfluss erhitzt und für 13 h gerührt. Nach Abkühlen auf Raumtemperatur wurde der Austrag über einer Filternutsche (Nennweite der Poren 10 bis 16 µm) filtriert. Man erhielt das Rohprodukt mit folgender Zusammensetzung: Isovaleraldehyd: 4,5 GC-Gew.-% (t_{R} = 3,6 min); Dihydropyrane (III.1 - III.3): 68,8 GC-Gew.-% (t_{R} = 9,4, 11,2, 11,7 min); Isoprenol: 0,4 GC-Gew.-% (t_{R} = 9,9 min); 4,4-Dimethyl-2-isobutyl-1,3-dioxan: 2,4 GC-Gew.-% (t_{R} = 11,4 min); 2-Isobutyl-4-hydroxy-4-methyl-tetrahydropyran: 19,5 GC-Gew.-% (t_{R} = 27,3 min, 28,6 min).

## Patentansprüche

1. Verfahren zur Herstellung von 2-substituierten 4-Methyl-tetrahydropyranen der
allgemeinen Formel (I) 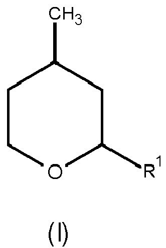 worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) ein Ausgangsmaterial bereitstellt, das wenigstens eine Dioxanverbindung
der allgemeinen Formel (II) enthält 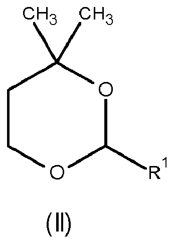 wobei R¹ die zuvor angegebene Bedeutung hat,
b) das Ausgangsmaterial einer Umsetzung in Gegenwart einer starken Säure und/oder eines sauren Ionenaustauschers unterzieht, wobei ein gegenüber dem Ausgangsmaterial an der Dioxanverbindung der Formel (II) abgereichertes und an wenigstens einer der Verbindungen der Formeln (III.1), (111.2) oder (111.3) 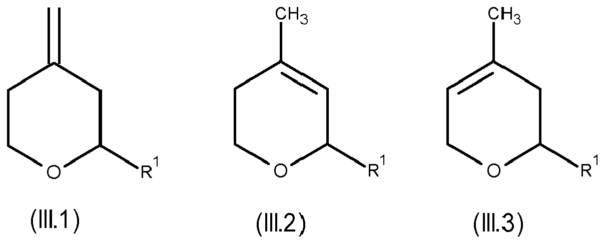 angereichertes Produktgemisch erhalten wird, wobei R¹ die zuvor angegebene Bedeutung hat,
c) das in Schritt b) erhaltene Produktgemisch einer Hydrierung unterzieht.

2. Verfahren nach Anspruch 1, wobei in Schritt a) ein Ausgangsmaterial bereitgestellt wird, das zusätzlich wenigstens eine der Verbindungen der Formeln (III.1), (111.2) oder (111.3) 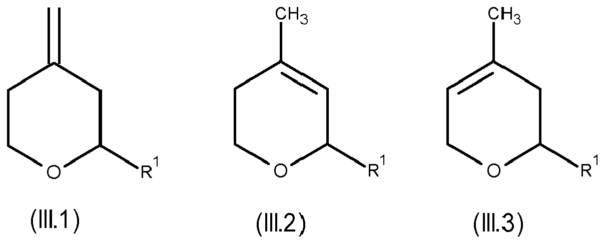 enthält, worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

3. Verfahren nach einem der vorhergehenden Ansprüche, wobei man zur Bereitstellung des Ausgangsmaterials in Schritt a):
a1) 3-Methylbut-3-en-1-ol der Formel (IV) 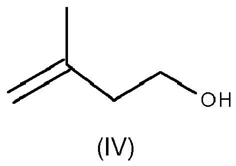 mit einem Aldehyd der Formel (V)
R¹-CHO (V)
worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
in Gegenwart eines sauren Katalysators umsetzt, wobei ein Reaktionsgemisch erhalten wird, das wenigstens eine Dioxanverbindung (II), wenigstens eine der Verbindungen (III.1), (III.2) oder (III.3) und wenigstens ein 2-substituiertes 4-Hydroxy-4-methyl-tetrahydropyran der allgemeinen Formel (VI) 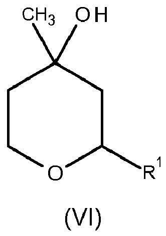 enthält, wobei R¹ in der Formel (VI) die zuvor angegebene Bedeutung hat,
a2) gegebenenfalls das Reaktionsgemisch aus Schritt a1) einer Auftrennung
unter Erhalt wenigstens einer an 2-substituierten 4-Hydroxy-4-methyl-tetrahydropyranen der allgemeinen Formel (VI) angereicherten Fraktion und einer Fraktion, die die Dioxanverbindung (II) und wenigstens eine der Verbindungen (III.1), (III.2) oder (III.3) enthält, unterzieht,
und das in Schritt a1) erhaltene Reaktionsprodukt oder die in Schritt a2) erhaltene Fraktion, die die Dioxanverbindung (II) und wenigstens eine der Verbindungen (III.1), (III.2) oder (III.3) enthält, als Ausgangsmaterial zur Herstellung der 2-substituierten 4-Methyl-tetrahydropyrane der Formel (I) einsetzt.

4. Verfahren nach Anspruch 3, wobei man in Schritt a2) das Reaktionsprodukt aus Schritt a1) einer destillativen Auftrennung unterzieht.

5. Verfahren nach Anspruch 4, wobei in Schritt a2) zur destillativen Auftrennung des Reaktionsprodukts aus Schritt a1) eine Anordnung von Destillationskolonnen eingesetzt wird, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst, und
a21) das Reaktionsgemisch aus Schritt a1) zunächst einer Auftrennung in der konventionellen Destillationskolonne unterzogen wird, wobei ein erstes Kopfprodukt erhalten wird, das an der Dioxanverbindung (II) und an den Verbindungen (III.1), (III.2) und (III.3) angereichert ist und im Wesentlichen keine Verbindungen der allgemeinen Formel (VI) enthält, und ein erstes Sumpfprodukt erhalten wird, das an den Verbindungen (III.1), (III.2) und (III.3) und der Dioxanverbindung (II) abgereichert ist und die Hauptmenge der Verbindungen der allgemeinen Formel (VI) enthält,
a22) das erste Sumpfprodukt aus Schritt a21) einer Auftrennung in der Trennwandkolonne oder in der Zusammenschaltung von zwei thermisch gekoppelten konventionellen Destillationskolonnen unterzogen wird, wobei ein zweites Kopfprodukt erhalten wird, das die nicht im ersten Kopfprodukt enthaltenen Verbindungen (III.1), (III.2), (III.3) und (II) sowie gegebenenfalls geringe Mengen der Verbindungen der allgemeinen Formel (VI) enthält, ein Seitenstrom erhalten wird, der im Wesentlichen aus Verbindung der allgemeinen Formel (VI) besteht und ein zweites Sumpfprodukt erhalten wird, das die Verbindungen der allgemeinen Formel (VI) enthält, die nicht im Kopfprodukt und nicht im Seitenstrom enthalten sind,
wobei das erste Kopfprodukt und/oder das zweite Kopfprodukt als Ausgangsmaterial in Schritt b) eingesetzt werden.

6. Verfahren nach einem der vorhergehenden Ansprüche, wobei der Rest R¹ für Isobutyl oder Phenyl steht.

7. Verfahren nach einem der vorhergehenden Ansprüche, bei dem die Umsetzung in Schritt a1) und/oder in Schritt b) in Gegenwart einer Säure erfolgt, die ausgewählt ist unter Salzsäure, Schwefelsäure, Methansulfonsäure, p-Toluolsulfonsäure und stark sauren Kationenaustauschern.

8. Verfahren nach Anspruch 7, bei dem die Umsetzung in Schritt a1) und/oder in Schritt b) in Gegenwart eines stark sauren Kationenaustauschers durchgeführt wird.

9. Verfahren nach einem der vorhergehend Ansprüche, wobei die Schritte b) und c) zumindest teilweise zeitgleich durchgeführt werden.

10. Verfahren nach einem der vorhergehenden Ansprüche, wobei die Hydrierung in Schritt c) in Gegenwart eines Hydrierkatalysators erfolgt, der ausgewählt ist unter homogenen und heterogenen Katalysatoren, die wenigstens eine Metallkomponente, ausgewählt unter Metallen, Metalloxiden, Metallverbindungen oder Gemische davon enthalten, insbesondere Palladium auf Kohlenstoff, Platin auf Kohlenstoff, Raney-Nickel oder Raney-Cobalt.

11. Verfahren nach einem der vorhergehenden Ansprüche, wobei das in Schritt c) erhaltene Hydrierprodukt einer destillativen Auftrennung unterzogen wird (= Schritt d).

12. Verfahren nach Anspruch 11, wobei in Schritt d) aus dem in Schritt c) erhaltenen Hydrierprodukt eine an 2-substituierten 4-Methyl-tetrahydropyranen (I) angereicherte Fraktion isoliert wird, die einen Gehalt an Dioxanverbindungen der allgemeinen Formel (II) von höchstens 2 Gew.-%, besonders bevorzugt von höchstens 1 Gew.-%, insbesondere von höchstens 0,5 Gew.-%, speziell von höchstens 0,1 Gew.-%, aufweist.

13. Verfahren nach Anspruch 12, wobei die in Schritt d) erhaltene an 2-substituierten 4-Methyl-tetrahydropyranen (I) angereicherte Fraktion wenigstens einem Waschschritt mit Wasser und/oder einer weiteren destillativen Aufreinigung unterzogen wird.

14. Verfahren nach einem der vorhergehenden Ansprüche zur Herstellung von 2-Isopropyl-4-methyl-tetrahydropyran (I).
